# EUROPÄISCHE PATENTSCHRIFT

(11) **EP 1 218 803 B1**
(45) Veröffentlichungstag und Bekanntmachung des Hinweises auf die Patenterteilung: **04.06.2003**
(21) Anmeldenummer: 00942037.3
(22) Anmeldetag: 08.06.2000
(51) Int. Cl.: G05B 19/18

(54) **VERFAHREN ZUM HUMPELDREHEN UND BEVORZUGTE ANWENDUNGEN DES VERFAHRENS**
HOBBLE TURNING METHOD AND PREFERRED APPLICATIONS OF SAID METHOD
PROCEDE DE TOURNAGE PAR SAUTS ET APPLICATIONS PREFEREES DUDIT PROCEDE

(30) Priorität: 08.06.1999 DE 19925924
(43) Veröffentlichungstag der Anmeldung: 03.07.2002
(73) Patentinhaber: Hörmansdörfer, Gerd, 31303 Burgdorf-Beinhorn (DE)
(72) Erfinder: Hörmansdörfer, Gerd, 31303 Burgdorf-Beinhorn (DE)
(74) Vertreter: Siekmann, Gunnar, Dipl.-Phys.
(86) Internationale Anmeldenummer: EP0005325
(87) Internationale Veröffentlichungsnummer: WO00075737

(56) Entgegenhaltungen:
- EP-A- 0 902 342
- WO-A-97/39702
- WO-A-99/33416
- BAR G ET AL: "SOFTWARE ZUM BERECHNEN UND KONSTRUIEREN VON SCHNECKEN UND GEWINDEN SOWIE IHRER WERKZEUGE" WERKSTATT UND BETRIEB,DE,CARL HANSER VERLAG. MUNCHEN, Bd. 122, Nr. 4, 1. April 1989 (1989-04-01), Seiten 289-291, XP000036681 ISSN: 0043-2792

## Beschreibung

Die Erfindung betrifft ein spezielles Verfahren zur drehtechnischen Bearbeitung von Werkstücken und bevorzugte Anwendungen des Verfahrens.

Im Prinzip ist die konventionelle Drehtechnik ein seit sehr langer Zeit bekanntes Verfahren für die spanende Herstellung von Werkstücken z.B. aus Holz, Metall oder Kunststoff. In jüngerer Zeit hat die Drehtechnologie durch die Einführung und Fortentwicklung numerischer Steuerungen eine rasante Ausweitung ihrer Möglichkeiten erfahren. So ist heute z.B. die Einhaltung einer konstanten Schnittgeschwindigkeit entlang der Oberflächenkontur überhaupt kein Problem. Selbst komplizierteste rotationssymmetrische Geometrien sind mittels einer entsprechenden Programmierung relativ einfach zu verwirklichen und in sehr kurzen Bearbeitungszeiten herstellbar. Daneben wurden derartige Maschinen durch die Ausrüstung mit Werkzeugantrieb weiter aufgewertet, weil so komplex geformte Werkstücke in einer Aufspannung dreh- und frästechnisch fertig bearbeitet werden können. Trotzdem bestehen hier gewisse Einschränkungen, welche entweder den Faktor Zeit oder bestimmte geometrische Gestaltungen betreffen. Es ist z.B. eine Tatsache, daß die drehtechnische Herstellung generell deutlich kürzere Bearbeitungszeiten ermöglicht als das Fräsen. Ausserdem ergeben sich beim Drehen bessere Oberflächenquatitäten. Wenn wegen der Werkstückgeometrie nur eine frästechnische Herstellung in Frage kommt, muß daher zwangsläufig eine deutlich längere Bearbeitungszeit bzw. eine ungleichmässigere Oberfläche in Kauf genommen werden. Aber auch mit einer frästechnischen Herstellung sind die geometrischen Möglichkeiten limitiert. So kann beispielsweise jede Ecke einer gefrästen Kontur in der Radialebene der Fräserochse niemals scharfkantiger sein als der Radius des verwendeten Fräsers. Scharfkantige Konturen sind zwar mittels Räumen, Stossen oder durch Erodieren erzielbar, jedoch muss dazu das Werkstück auf eine andere Maschine übernommen werden. Im Falle des Erodierens ist der Zeitbedarf extrem hoch. Zwar sind für die spanende Herstellung unrunder Konturen seit einigen Jahren sogenannte Formbohr- bzw. Formdrehgeräte auf dem Markt, jedoch hoben diese Geräte ihren Preis und erfordern so eine kapitalmäßige Investition entsprechender Grössenordnung. Außerdem sind sie nur an die vorgesehene Schnittstelle anschliessbar und auf eine vorgegebene Kontur mit zweidimensionaler Unrundheit beschränkt.

Bereits früher hatte es Versuche gegeben, Drehmaschinen durch Anbau spezieller mechanischer Baugruppen für die Bearbeitung von unrunden Werkstöcken zu ertüchtigen. Eine entsprechende Maschine wird in der Deutschen Offenlegungsschrift DE 25 15 106 vorgeschlagen. Neben dem sehr aufwendigen und anfälligen Bauaufwand krankt diese Maschine an der extremen Limitierung ihrer Möglichkeiten, welche sich ohnehin nur auf die Erzeugung einer zweidimensionalen Unrundgeometrie beschränken.

Die geometrischen Möglichkeiten der Unrundbearbeitung sind bezüglich auf eine Drehmaschine aufrüstbarer Werkzeuge z.B. erweiterbar, wenn der Schneidenantrieb frei programmierbor angesteuert werden kann. Ein solches Werkzeug ist z.B. aus der Deutschen Offenlegungsschrift DE 35 09 240 A1 bekannt. Hier werden piezoelektrische oder magnetostriktive Stellglieder herangezogen, um eine dynamische Schneidenverschiebung relativ zum Werkstück mittels einer entsprechenden elektrischen Ansteuerung zu verwirklichen. Hiermit sind jedoch nur sehr kleine Stellwege erzielbar. Es wäre zwar technisch möglich, z.B. durch Anwendung eines magnetodynamischen Systems zu wesentlich größeren Stellwegen zu gelangen, doch wären diese wie zuvor auf eine einzige Bewegungsachse limitiert. Zur Erzielung bestimmter dreidimensionaler Unstetbearbeitungen wäre es notwendig, durch Hinzufügen einer zweiten oder sogar dritten jeweils orthogonal angeordneten Bewegungseinheit ein Werkzeug mit komplexen Bewegungsrichtungen zu schaffen, allerdings wäre dieses aufwendig im Bau und anspruchsvoll in Bezug auf die erforderliche Ansteuerelektronik. Ein derartiges Werkzeug ist bisher nicht verfügbar.

Es sind auch spezielle Drehmaschinen bekannt, welche für die Unrundbearbeitung z.B. von Kolben für Verbrennungsmotoren entwickelt worden sind. Moderne Kolben besitzen nämlich einen leicht unrunden, in der Regel elliptischen Querschnitt, um die anisotrope Ausdehnung bei Erwärmung zu kompensieren. Allerdings besteht hier nur eine sehr geringe Abweichung von der Kreisform, wobei die Kontur außerdem einen weich fliessenden Verlauf aufweist. Sprünge oder extreme Unstetigkeiten sind dort nicht vorhanden. Demgemäss besteht hinsichtlich der baulichen Auslegung einer derartigen Maschine kein sehr hoher Schwierigkeitsgrad. Es genügt im Prinzip, den Drehmeissel in der den Durchmesser betreffenden X-Achse mit geringer Amplitude schwingen zu lassen, während der Schlitten entlang dem Werkstück in der Z-Achse verfahren wird. Dabei wird die Auslenkungskurve der Drehmeisselspitze einen mehr oder weniger sinusförmigen Verlauf zeigen, sodaß extreme Beschleunigungen gar nicht erforderlich sind. Diese wären trotz der reduzierten Masse des Systems ohnehin nur schwer zu realisieren. Es versteht sich, daß derartige Maschinen eine Verkopplung der Werkstückrotation zur Bewegung der X-Achse erfordern, jedoch ist der Vorschub in der Z-Achse frei gestaltbar. Tatsächlich ist die Erzeugung der unrunden Kontur dabei auf die zweidimensionole Durchmesserebene beschränkt und wird mittels der Z-Achse auf die dritte Dimension lediglich ausgedehnt. Die Z-Achse ist dabei jedoch in die unrunde Konturerzeugung nicht wirklich einbezogen. Ein Verfahren des Schlittens entlang der Z-Achse in Sprüngen oder mit einer etwa überlagerten Oszillation ist nicht vorgesehen.

Eine Sondermaschine der oben genannten Art wird z.B. in der Deutschen Offenlegungsschrift DE 40 31 079 A1 beschrieben, wobei vorgeschlagen wird, für die Ansteuerung des für die oszillierende Bewegung des Drehmeissels vorgesehenen Antriebs (z.B. elektrischer Linearmotor oder hydraulisches System) neben der vorhandenen Maschinensteuerung eine zusätzliche Rechnersteuerung z.B. in Gestalt eines Personalcomputers heranzuziehen. Ohne Abänderung des zugrunde liegenden kinematischen Verfahrens ist jedoch eine derartige Maschine in ihren Möglichkeiten auf die vorgesehene und ähnliche Anwendungen beschränkt. Außerdem ist eine solche Sondermaschine in der Anschaffung verhältnismäßig teuer.

Es bestand daher die Aufgabe zur Schaffung eines Verfahrens für die drehtechnische Bearbeitung von Werkstöcken mit Unregelmäßigkeiten oder Unstetigkeiten der Kontur, welches einerseits die an der Maschine bestehenden Gegebenheiten hinsichtlich des Kreuzschlittens und der NC-Steuerung nutzen, ohne Zusatzgerätschaften auskommen, die mit der Massenträgheit einhergehenden Plobleme überwinden und gleichzeitig den Freiheitsgrad bezüglich der Unstetigkeit der erzeugbaren Kontur um wenigstens eine zusätzliche Dimension erweitem sollte. Dabei war auch angestrebt, bisherige frästechnische Operationen durch das neue Verfahren so weit wie möglich abzulösen.

Die genannte Aufgabe wird nach der Erfindung durch ein vom Anmelder als Humpeldrehen bezeichnetes drehtechnisches Verfahren gelöst, wobei dos Werkstück im Futter der Maschinenspindel mit einer - vorzugsweise konstonten - Drehzahl rotiert, und dabei der Kreuzschlitten mit dem Zerspanungswerkzeug unter Benutzung z.B. einer Gewinde- oder C-Achsen-Programmierung in der Steigungsachse synchronisiert zum Spindelwinkel verfahren wird und bestimmte unrunde, aus geometrischen Übergangselementen zusammengesetzte Konturen mittels einer Programmierung aus Sprungfunktionen durch Verknüpfung von Befehlssätzen mit Werten für die Adressparameter Durchmesser (X), Länge (Z) und entweder Winkel (C) oder Steigung (F) erzeugt werden, wobei mindestens für einen dieser Parameter in der Programmsatzkette eine Folge aus humpelnden Wertegruppen mit mindestens einem Zahlenwert in jeder Wertegruppe verwendet wird. Das Verfahren ist durch Heranziehung des Parameters Y (Höhe) bei entsprechend ausgerüsteten Maschinen erweiterbar.

Die bei den meisten Bearbeitungsaufgaben in der Programmsatzkette für mindestens einen Adressparameter zwischen den Zahlenwerten gebildeten Inkremente stellen eine humpelnde Folge aus Wertegruppen mit mindestens einem Zahlenwert in jeder Wertegruppe dar, wobei z.B. die entsprechenden Zahlenwerte innerhalb der einen Wertegruppe grösser sind als die innerholb der anderen und/oder das Vorzeichen innerhalb der einen Wertegruppe positiv und innerhalb der anderen Wertegruppe negativ ist. Im Prinzip bilden die für einen bestimmten Adressparameter programmierten Werte in der Programmsatzkette eine Folge von Zohlenwerten, in welcher die befohlenen Sprungfunktionen als sogenannte Humpeischritte zum Ausdruck kommen.

Besondere Bedeutung erlangt das Verfahren durch seine Anwendungsmöglichkeit in allen drei Dimensionen, selbst ohne Heranziehung der Y-Achse. Diese bearbeitungsmässige Freiheit ist darauf zurückzuführen, daß Humpetschritie mittels der Progrommparameter X, Z, F und C jeweils allein oder in Kombinotion miteinander programmierbar sind.

Das Verfahren wird erfindungsgemäss durch ein Sprungsystem erweitert, wobei die herzustellenden Unstetigkeiten in aufeinander folgenden Sequenzen aus geornetrisch gegeneinander versetzten Drehzyklen erzeugt werden.

Das erfindungsgemässe Verfahren benötigt weder Spezialgerätschaften noch zusätzliche NC-Steuerungen und beruht allein auf der Anwendung der mit der Maschinensteuerung und der entsprechenden Software gegebenen Möglichkeiten und wird lediglich begrenzt durch die Dynamik des Gesamtsystems. Hierzu sind z.B. die bekannten Befehlssätze G 01, G 31, G 33, G 34, G 37 bzw. G 131 usw., sowie z.B. die Adressparameter Durchmessermass (X), Längenmaß (Z), Gewindesteigung (F), Anlauflänge (B), Überlauflänge (P), Spindelwinkel (C), Bezugsrichtung für F (H) und Steigungsänderung (E) verwendbar oder einfügbare Blöcke mit individueller Software. Es ist auch nicht ausgeschlossen, dass aufgrund des hier vorgeschlagenen Verfahrens in der Zukunft erweiterte Programmierungsmöglichkeiten seitens der Industrie serienmäßig angeboten werden.

Die oben angesprochene Dynamik des Gesamtsystems setzt sich zusammen aus der mechanischen und elektronischen Dynamik der Maschine. Dabei ist die mechanische Dynamik abhängig von der Masse des Kreuzschlittens und der Reaktionsgeschwindigkeit des Antriebs z.B. aus Gewindespindeln, Motoren und Getriebe. Demgegenüber ist die elektronische Dynamik durch die Rechengeschwindigkeit der Steuerung und deren Verknüpfungssteifigkeit mit den elektromotorischen Antrieben vorgegeben. Demgemäss sind Drehmaschinen der allerneuesten Generation mit digitalen Antrieben und schnellsten Rechnern für extreme Unstetbearbeitungen geeignet, während die Anwendung des Verfahrens auf älteren Maschinen entsprechend eingeschränkt ist. Diese Beschränkung kann teilweise durch die Benutzung reduzierter Schnittgeschwindigkeiten während der Zerspanung zurückgedrängt werden, weil sich daraus niedrigere Spindeldrehzahlen und entsprechend reduzierte Vorschubgeschwindigkeiten ergeben.

Eine sehr einfache Anwendung des Verfahrens besteht z.B. in der drehtechnischen Herstellung exzentrischer Zapfen. Hierzu wird mittels einer Verkettung von Befehlssätzen z.B. mit G 33 eine in Bezug auf das Werkstück drehwinkelmäßige Auflösung von 180° realisiert, indem eine Koordinotenkette aus jeweiligen Zahlenwerten für X und Z sowie eine Steigung in F programmiert werden, wobei die zwischen den für den genannten Winkelschritt von jeweils 180° in Z programmierten Werten liegenden Inkremente im Prinzip dem halben programmierten Steigungswert entsprechen müssen. Demgegenüber springen die Werte für X bei jedem 180°-Halbschritt zwischen einem grösseren und einem kleineren programmierten Durchmesserwert hin und her, wobei theoretisch der Mittelwert dem Durchmesser, und die holbe Differenz der Exzentrizität des herzustellenden Zapfens entsprechen. Zwecks Vereinfachung des Programmieraufwandes können die z.B. in der Längs- bzw. Durchmesserachse sich wiederholenden Sprünge bei einigen Steuerungen als sogenannte Variable eingegeben werden. Da für das beschriebene Bearbeitungsbeispiel die Durchmesseränderung in der Regel grösser als der beabsichtigte Vorschub in Gestalt der Steigung ist, wird die Maschinensteuerung im Normalfall die programmierte Steigung mit dem Vorschub der X-Achse verrechnen. Daher muß für die Steigung unter F der bezüglich des Durchmessers pro Umdrehung programmierte Weg, also die doppelte Durchmesserdifferenz, eingegeben werden, wenn nicht der Umsprung durch Befehlssätze z.B. mit H unterbunden wird. Aus der beschriebenen Programmierung ergibt sich eine theoretische Bahnkurve des Kreuzschlittens in Gestalt einer fortlaufenden Zick-Zack-Linie. Tatsächlich wird aufgrund der verschiedenen dämpfend wirkenden Faktoren, wie z.B. hoher Kreuzschlittenmasse und ungenügender Steifigkeit des Regelkreises, ein sich ständig wiederholender quasi sinusartiger Bewegungsablauf des Kreuzschlittens während des Vorschubs entlang des Werkstücks erzielt, sodass trotz einer im Prinzip primitiven Progrommierung eine erstaunliche Rundheit des exzentrischen Zapfens resultiert. Andererseits ergibt sich aus dieser Verzerrung, daß die am Werkstück nachmessbaren Abmessungen nicht exakt den programmierten Werten entsprechen. Daher müssen die zu programmierenden Zahlenwerte anhand von Probestücken ermittelt werden. Danach sind diese allerdings mit hoher Genauigkeit auf der jeweiligen Maschine reproduzierbar.

Die oben beschriebene Vorgehensweise ist für die drehtechnische Herstellung elliptischer Körper abwandelbar, indem die programmierte Zick-Zack-Kurve mit doppelter Auflösung, also mit drehwinkelmäßigen Schritten von 90°, festgelegt wird. Nun beschreiben die beiden alternierend programmierten Durchmesser den theoretischen Grösst- bzw. Kleinstdurchmesser der Ellipse. Die dann gewöhnlich von der Steuerung in der X-Achse verrechnete Steigung muß dann mit der vierfachen Durchmesserdifferenz programmiert werden.

In entsprechender Weise wird vorgegangen, wenn ein Polygon (sogenanntes Gleichdick) hergestellt werden soll, wobei eine Auflösung des Winkelschritts von 60° erforderlich ist. Eine derartige Bearbeitung ist z.B. in Gestalt einer planseitig eingestochenen Nut interessant, wie sie heute z.B. als Schmiemut von Anlaufscheiben oder Reinigungsnut an Bremsscheiben bekannt ist. Bei den genannten Beispielen ist eine exakt beschriebene Nutenbahn für die ordnungsgemässe Funktion nicht erforderlich, sodass eventuelle Bahnabweichungen bedeutungslos sind.

Bei den oben beschriebenen Beispielen handelt es sich um relativ harmonische Unrundbearbeitungen mit konstantem Vorschub in der Längsachse bei fest programmierter Steigung. Es ist ohne weiteres möglich, die beschriebene Programmierung durch Einfügen von Hilfspunkten zu erweitern und so zu einer perfektionierten Kontur zu gelangen. Das erfindungsgemässe Verfahren geht hier jedoch noch weiter, indem für die spanende Herstellung von Werkstücken mit grösserer ünstetigkeit bzw. Eckigkeit der Kontur, oder der Realisierung einer höheren Bahngenauigkeit die Heranziehung von variierenden Steigungswerten - z.B. auch in Verbindung mit einer feineren Auflösung der Kontur - vorgeschlagen wird. Im Progromm wird die zur Erzielung einer bestimmten Kontur vom Kreuzschlitten abzufahrende Bahn dann aus verketteten Sätzen z.B. mit G 33 beschrieben und für jeden Programmsatz eine andere Steigung festgelegt, wobei im Extremfall z.B. ein erster Programmsalz einen sehr kleinen, bzw. ein nächster Programmsatz einen sehr großen Wert für F aufweist, usw., so dass z.B. eine Abfolge aus weichen und ruckartigen Bewegungen des Kreuzschlittens entsteht. Mit diesem Verfahren sind drehtechnische Unstetbearbeitungen in einer grossen Vielfalt realisierbar, z.B. auch auf den Mantelflächen gekrümmter Körper.

In gleicher Weise ist mittels des Verfahrens die in den Programmsätzen abgelegte Koordinatenkette aus jeweiligen X- und Z-Werten für sich allein, oder auch in Verbindung mit springenden F-Werten heranziehbar, um derartige unstete Konturverläufe zu verwirklichen. So ist z.B. der Vorschub einer oder beider Achsen als sogenannter Pilgerschritt programmierbar, wobei nach einer bestimmten Vorschubstrecke jeweils ein z.B. ruckartiger (kürzerer) Rücksprung folgt, dem wiederum eine z.B. grössere Vorschubstrecke nachgeschaltet ist. Sinngemäss kann eine derartige Bearbeitung z.B. als das wechselweise Schneiden von verketteten Rechts- und Linksgewinden mit unter Umständen unsymmetrischer Gewindesteigung aufgefasst werden.

Das erfindungsgemässe Verfahren erlaubt auch die spanende Herstellung unstet verlaufender, aus einer geneigten oder gekrümmten Mantelfläche herausstehender Konturelemente, wobei mit der Seite des Drehmeissels im wesentlichen die Flanke des unstet verlaufenden Konturelements und mit der Spitze des Drehmeissels im wesentlichen die Mantelfläche bearbeitet wird. Hierbei wird durch entsprechende Programmierung von Startund Zielpunkten sowie Steigung die Spitze des Drehmeissels auf einer im wesentlichen auf der Mantelfläche verlaufenden Bahn geführt und mit der Seite des Drehmeissels mittels einer programmierten Änderung der Verfahrgeschwindigkeit und/oder Verfahrrichtung die Flanke des unstet verlaufenden Konturelements erzeugt.

Bei der beschriebenen Programmierung ist insbesondere darauf zu achten, daß die gewöhnlich mit dem Adreßparameter H bezeichnete Bezugsrichtung für F richtig verwendet wird. Bekanntlich wird unter H festgelegt, mit welcher Achse der Vorschub verrechnet wird, welcher der unter F programmierten Gewindesteigung entspricht. Ohne Angabe oder mit H=0 bezieht sich der Vorschub auf die Z-Achse, also im Prinzip auf Längs-, Kegel- und entsprechende verkettete Gewinde bis maximal 45° zur Z-Achse. Wird H auf 1 gesetzt, so betrifft die Vorschubverrechnung nun die X-Achse, also grundsätzlich Plan-, Kegel- und entsprechende verkettete Gewinde bis maximal 45° zur X-Achse. Daneben ist mit H=3 der Vorschub auf die Gewindebahn beziehbar. Bei verketteten Gewinden auf gekrümmten Flächen kann es leicht vorkommen, daß der Grenzwert von 45° überschritten wird und die Maschinensteuerung dann automatisch auf die andere Achsenverrechnung umspringt. Entweder muss diese dann z.B. durch Umrechnung ermittelt und im Programm bewusst verfälscht angegeben sein, oder es muss der Umsprung per Software unterbunden werden, falls die Steuerung einen entsprechenden Befehlssatz bereit holt, z.B. mit 1 für eine Planund K für eine Längssteigung.

Daneben besteht bei der Programmierung der Zielkoordinaten X und Z in Verbindung mit der Steigung F unter einem Befehlssatz für Gewinde (z.B. G33) das Problem, daß eine real vorkommende Steigung von Null von der Steuerung nicht akzeptiert wird. Eine Möglichkeit zur Überwindung dieses Hemmnisses besteht dann darin, diesen Parameter auf das kleinste programmierbare Inkrement (z.B. 0,001 mm) zu setzen.

Mit der Erfindung wird jedoch eine noch elegantere Methode zur Eliminierung dieses Problems zur Verfügung gestellt, wobei diese gleichzeitig noch sowohl den Umsprung bei 45° umgeht, als auch den Programmieraufwand reduziert. Danach wird das Humpelprogramm z.B. unter dem Befehlssatz G01 durch Koordinatenketten aus X und Z gebildet und unter C der jeweilige Spindelwinkel angegeben. Eine Berechnung der jeweiligen Steigung entfällt dann, da sich diese aus den Differenzen des im jeweiligen Fall gewählten Bezugsparameters (Z oder X) im Verhältnis zum Spindelwinkel C ergibt. Wenn dann die Winkelschritte zwischen den in den Programmsätzen folgenden Spindelwinkeln gleich sind oder sich in einer bestimmten Regelmäßigkeit z.B. als Humpetrythmus wiederholen, so kann der Wert für C als Variable programmiert werden. Dann wird dieser Parameter in seinem Wert nach der Abarbeitung des jeweiligen Programmsatzes um die ebenfalls als Variable oder Festwerte programmierbaren jeweiligen Winkelschrittwerte erhöht oder emiedrigt. Falls eine Änderung des unter Umständen sehr langen Programms erforderlich sein sollte, genügt dann in der Regel das Überschreiben weniger Festwerte oder Variablen.

Das oben vorgeschlagene Verfahren der Spindelwinkelprogrammierung ist allerdings nur bei bestimmten Maschinen und NC-Steuerungen möglich, die dem neuesten Entwicklungsstand entsprechen. Hier ist maschinenseitig die Spindel in den Antriebsmotor integriert, wobei die ganze Einheit sowohl als Drehachse als auch als C-Achse ansteuerbar ist. Bei einer entsprechend schnellen NC-Steuerung besteht dann hinsichtlich der Programmierung eine gewisse Äquivalenz hinsichtlich der Drehgeschwindigkeit der Spindel, welche z.B. darin zum Ausdruck kommt, daß die C-Achse bis zu hohen Drehzahlen (u.U. mehrere tausend Umdrehungen pro Minute) benutzbar ist. Damit sind mittels der Programmierung der C-Achse Schnittgeschwindigkeiten realisierbar, die denen der üblichen Drehoperationen entsprechen.

Das gesamte erfindungsgemässe Verfahren wird noch durch den Vorschlag erweitert, die aufgrund der limitierten Maschinendynamik bestehenden Anwendungsgrenzen dadurch zu überwinden, daß für extreme Bearbeitungsgeometrien eine Verschachtetung von Bearbeitungssequenzen herangezogen wird. Dabei handelt es sich um eine Art Sprungverfahren, welches in einem ersten Bearbeitungszyklus z.B. ein erstes Konturelement bearbeitet, dabei jedoch ein zweites auslässt, um mit einer beruhigten Bahn wiederum ein drittes Konturelement abzufahren, usw... . Die beim ersten Bearbeitungszyklus ausgelassenen Konturelemente werden in einem zweiten Bearbeitungszyklus zerspant, wobei nunmehr die Konturelemente aus dem ersten Bearbeitungszyklus ausgelassen werden. Dieses Verfahren berücksichtigt das aus einer mit maximaler Verfahrgeschwindigkeit programmierten abrupten Bewegung resultierende Überschwingen des Gesamtsystems, welches ein in kurzer Entfernung folgendes Konturelement nicht in der gewünschten Weise abzufahren in der Lage ist. Zwecks Ausführung des Verfahrens ist damit wegen der z.B. zwei oder mehr Bearbeitungssequenzen zwar ein höherer Zeitaufwand erforderlich, jedoch ist dieser gegenüber einer frästechnischen Herstellung immer noch drastisch kürzer.

Mit der Erfindung werden gleichzeitig bevorzugte Anwendungen des Verfahrens vorgeschlagen. Diese Anwendungen sollen anhand von Ausführungsbeispielen gleichzeitig einer näheren Erläuterung des Verfahrens dienen.

Eine der vorgeschlagenen Anwendungen betrifft die Gewindeherstellung verschiedener, insbesondere selbstschneidend in nachgiebiges Material eindrehbarer Einschraubkörper, wie z.B. Holz-, Kunststoff- oder Knochenschrauben, darunter z.B. Implantate wie Schenkelhalsschrauben, Fusionskörper, Schrauben für den so genannten Fixateur Externe, Eindrehpfosten für Zahnimplantate, oder künstliche Hüftgelenkpfannen.

Eine andere Anwendung betrifft die kostengünstige Herstellung von so genannten Kreiskeilprofilen an den inneren oder äusseren Kupplungsflächen von Verbindungselementen im Maschinenbau.

Eine der oben vorgeschlagenen Anwendungen bezieht sich auf vorzugsweise selbstschneidend einschraubbare künstliche Hüftgelenkpfannen, welche für die so genannte zementfreie Implantation beim Menschen vorgesehen sind. Derartige Schraubpfannen sind in den unterschiedlichsten Ausführungen am Markt. Für eine zuverlässige und dauerhafte Integration und auch eine vorteilhafte Handhabbarkeit beim implantieren ist die Gestaltung des Gewindes von ausschlaggebender Bedeutung. Es ist mittlerweile bekannt, dass eine grosse Kontaldftäche des Implantats zum knöchernen Lager ohne Lastspitzen und ein zum Pfannenpol geneigtes Gewindeprofil gute Voraussetzungen für die Vermeidung von Lockerungen sind. Daneben muß eine solche Schraubpfanne eine gute Taktilianz besitzen, womit die während des Einschraubens von der Schraubpfanne vermittelte Fühlbarkeit des Aufsetzens des Schalenkörpers auf die vorbereitete knöcherne Aufnahmefläche im Acetabulum bezeichnet wird. Bei den bisherigen Schraubpfannentypen besteht hier Handlungsbedarf, weil bei Ihnen nach dem Implantieren entweder unerwünschte Freiräume zur knöchernen Grenzfläche hin bestehen, oder sie nur mit großer Kraftanstrengung eingeschraubt werden können, bzw. ihre Taktilianz unzureichend ist.

Eine Gruppe von Schraubpfannen ist mit einem sogenannten Flachgewinde versehen, bei welchem die seitlichen Flächen der Gewinderippe parallel zueinander stehen. Es ist üblich, die Gewinderippen zwecks Bildung von Schneidkanten in bestimmten Abständen durch das Einbringen von Spannuten zu unterbrechen. Bei dieser Gewindeart muss die Schneidkraft beim selbstschneidenden Einschrauben vollständig von der radial nach aussen zeigenden Kopffläche der Gewinderippe bzw. den dortigen Schneidkanten erbracht werden. Ohne weitere Maßnahme beschreibt jedoch der durch die Kopffläche der einzelnen Gewindeflügel repräsentierte Kurvenzug in der axialen Aufsicht von der Polseite der Schraubpfanne her eine Spirale, deren genaue Bahn von der Formgestalt des Schalenkörpers der Schraubpfanne und der Gewindesteigung abhängt. Dadurch nimmt mit fortschreitender Windung der radiale Kurvenabstand von der axialen Mittellinie zu. Das Ende eines jeden Gewindeflügels steht daher radial weiter nach aussen heraus als sein Anfang. Auf diese Weise entsteht beim Einschrauben einer derartigen Schraubpfanne ein Klemmeffekt, welcher lediglich durch die von der aufgerauhten Oberfläche des Implantats auf das knöcherne Material wirkenden Raspelkräfte gemildert wird. Daher sind derartige Implantate mit einem unnötig hohen Einschraubkroftbedarf behaftet.

Andererseits sind Schraubpfannen mit Flachgewinde bekannt, deren Gewindeflügel durch gruppenweises Überfräsen mit einem Freiwinkel versehen sind. Allerdings ergeben sich aus der gewählten Bearbeitungsart gerade kopfseitige Flächen, welche als Sehnen gegenüber dem von der jeweiligen Schneidkante gebildeten Schwenkkreis zurückversetzt verlaufen. Dadurch sind Schraubpfannen mit einem solchen Gewinde zwar etwas leichter einschraubbar, besitzen jedoch wegen der verkürzten Gewindezohnhöhe nur eine reduzierte Fläche zur Übertragung von Kräften. Sehr nachteilig ist insbesondere die Spaltenbildung im Bereich des Gewindezahnkopfes zwischen Implontat und Knochen, sowie die auf das knöcherne Substrat wirkende Hebelwirkung wegen der zu tief geschnittenen Zahnrillen. Daher halten auch solche Schroubpfannen einer kritischen Betrachtung aus rein medizinischer Sicht nicht stand.

Schraubpfannen der oben beschriebenen Art mit Rachgewinde konnten bislang nur einen gewissen Marktanteil erobern. Gegenwärtig scheinen Schraubpfannen mit sogenanntem Spitzgewinde weiter verbreitet zu sein. Doch auch bei dieser Gruppe besteht prinzipiell der zuvor beschriebene Problemkomplex hinsichtlich des unakzeptablen Einschraubverhaltens und der Spaltenbildung in der Kontakizone. Die verschiedenen Versuche zur Reduzierung des Einschraubkraftbedarfs haben nämlich unter anderem dazu geführt, die eingefrästen Spannuten zu Lasten der Gewindeflügel sehr breit auszulegen. Dadurch ging wertvolle Kontaktfläche verloren, verbunden mit der Bildung ausgedehnter Hohlräume bzw. von der Kraftübertragung ausgeschlossener ossärer Bereiche.

Mit dem US-Patent 4,997,447 wird eine Schraubpfanne mit runden Gewinderillen vorgeschlagen, deren Kopfflächen der einzelnen Gewindeflügel bogenförmig verlaufen, wobei ein Freiwinkel dadurch realisiert ist, daß der vom Pfannenpol ausgehende Radius dieses Bogens mit zunehmender Entfernung von der Schneidkante immer kleiner wird. Bei dieser Schraubpfanne dürfte ohne Einbußen ihres guten Einschraubverhaltens das Ausmass der Spaltenbildung gegenüber geradlinigen Kopfflächen merklich reduziert sein. Allerdings ist für ihre Fertigung bisher ein recht hoher Zeitbedarf zu veranschlagen, da die vorgeschlagene Gestaltung das vollständige Abfahren der Zahnkopferstreckung mit einem Fräser erfordert.

Bisher sind in Bezug auf Schraubpfannen mit Spitzgewinde keine Ausführungen mit einem Freiwinkel der einzelnen Gewindesegmente auf dem Markt erschienen. Dies wird vermutlich damit zusammenhängen, daß eine entsprechende Realisierung mit einem hohen Schwierigkeitsgrad behaftet ist, und die sich zunächst anbietende frästechnische Herstellung neben einer sehr aufwendigen Programmierung einen sehr hohen zeitlichen Bearbeitungsaufwand erfordert. Diese Erschwernisse sind darin begründet, daß bei Spitzgewinden je nach Verlauf der Spannuten mindestens eine der Seitenflächen des Gewindezahns zur Bildung einer Schneidkante herangezogen werden muss. Wenn nun hinter der Schneidkante ein Neutral- oder Freiwinkel gebildet werden soll, so muss die entsprechende Seitenfläche des jeweiligen Gewindeflügels bis zur nächstfolgenden Spannut mit einem kongruenten Seitenwinkel hinterfräst werden. Dabei taucht das Problem auf, daß der Fräser bei gekrümmten Mantelflächen nicht gleichzeitig den Grund der Gewinderille konturtreu bearbeiten kann. Man hat dann die Wahl, entweder entlang der Zahnflanke eine immer mehr zunehmende rillenartige Vertiefung in Kauf zu nehmen, oder ein entsprechend anwachsendes treppenstufenartiges Relikt. Wenn dieses Überbleibsel nicht akzeptiert wird, muss es im Anschluß mittels mindestens eines weiteren Fräsganges entfernt werden.

Mit dem erfindungsgemäßen Verfohren ist es jetzt möglich, derartige Gewinde von Hüftgelenkpfonnen drehtechnisch in kürzester Zeit und mit Perfektion herzustellen. Dabei spielt es keine Rolle, ob die Unstetbearbeitung zur Erzeugung eines bestimmten Verlaufs der einzelnen Gewindeflügel an deren pol-, äquator- oder kopfseitiger Fläche bzw. mehreren dieser Flächen erfolgen soll. Wegen der freien Programmierbarkeit der Bearbeitungsbahn ist nicht nur jedes beliebige Profil des Gewindezahns beherrschbar, sondern auch der jeweilige winkelmäßige Verlauf der erzeugten Gewinderippenabschnitte nahezu frei bestimmbar. Gleichzeitig ist die gesamte Gewindeabwicklung an den Schalenmantel des Pfannenkörpers perfekt anpassbar. Daher kann die Erfindung für sämtliche bekannten Schalenformen wie z.B. sphärisch, asphärisch, paraphärisch, konisch-sphärisch, konisch, zylindrisch, parabolisch, toroidisch, usw. angewandt werden.

Das erfindungsgemässe Verfahren ist problemlos mit anderen bekannten Verfahren zur Herstellung von Gewinden für Hüftgelenkpfannen kombinierbar, z.B. mit dem aus der Europäischen Patentschrift EP 0 480 551 bekannten Verfahren, bzw. dem mit der Deutschen Offenlegungsschrift DE 44 00 001 vorgeschlagenen Verfahren zur Herstellung eines Gewindes mit veränderlich modifizierbarem Gewindeprofil. Besonders vorteilhaft erscheint eine Kombination mit einem zum Pfannenpol überkippten Gewindezahnprofil und einer sich fliessend ändernden Gewindesteigung gemäß der Internationalen Patentanmeldung WO 97/39702.

Diesbezüglich wird mit der Erfindung vorgeschlagen, bei künstlichen Hüftgelenkpfannen mit einem sich zum Kopf des Gewindezahns hin verjüngenden Zahnprofil die zwischen den Spannuten gebildeten Gewindeflügel jeweils aus so genannten Schraubflächen (auch bezeichnet als Schraubenflächen) zu bilden und wahlweise mit ihrer jeweiligen Erstreckungsrichtung in Abhängigkeit vom Drallwinkel der Spannut zu verschwenken. Als Schraubflächen werden dabei solche Flächen verstanden, welche durch Rotation eines bestimmten Zahnprofils mit konstantem radialen Abstand von der Pfannenachse und einer Steigung um dieselbe erzeugt sind. Bei einem z.B. trapezförmigen Zahnprofil sind folglich drei Schraubflächen gebildet, eine als kopfseitige und zwei als Seitenflächen. Dabei können diese Schraubflächen in ihrem Fussbereich entlang ihrer Erstreckung einer höhenmässigen Verkürzung unterliegen, wenn das Zahnprofil bei bestimmten Mantelgeometrien der Schraubpfanne in die Mantelfläche hinein verläuft. Die der Schneide am Anfang des jeweiligen Gewindeflügels folgenden Flächen besitzen dann einen Neutralwinkel, also weder einen Klemm- noch einen Freiwinkel. Dadurch sind unerwünschte Klemmeffekte beseitigt und trotzdem ein allseitiger Knochenkontakt der Gewindeflügel sichergestellt. Damit die am Anfang des jeweiligen Gewindeflügels vorhandene Schneide ihre Wirkung optimal entfalten kann, muß sie gegenüber dem vorauslaufenden Gewindeflügel vorstehen. Dies wird im ersten Schritt dadurch erreicht, dass für die Schraubflöchen eines nachfolgenden Gewindeflügels ein grösserer Radius herangezogen wird als für die Schraubflächen des vorauslaufenden Gewindeflügels. Vorzugsweise werden die einzelnen Gewindeflügel in ihrer Erstreckung in Abhängigkeit vom Drallwinkel der Spannuten relativ zueinander verschwenkt, wobei eine an den Drallwinkel sich annähernde Verschwenkungsrichtung bevorzugt ist, um einen Überstand der seitlichen Schneidkante mit positivem Spanwinkel zu realisieren.

Eine andere praktische Umsetzung der Erfindung besteht darin, bei der Herstellung derartiger Gewinde an festgelegten Positionen des Gewindezuges mittels der Programmierung von Humpelsprüngen überschwingende Übergangsfunktionen der Schneidenbahn zu erzeugen und diese mit den Spannuten so zu synchronisieren, dass die jeweils der Sponnut in Einschraubrichtung nachfolgende Schneidkante gegenüber dem Zahnprofil vorsteht. Der restliche Bereich des Zahnflügels weicht demgemäss gegenüber der Schneidkante zurück, sodass hinter der Schneidkante ein freiwinkelartiger Bereich gebildet ist.

Eine weitere Anwendung der Erfindung betrifft so genannte Kreiskeil- oder 3K-Kupplungen im allgemeinen Maschinenbau. Hierbei handelt es sich um reibschlüssige Dehnverbindungen z.B. zwischen Welle und Nabe, welche selbsthemmende, aber wieder lösbare Verbindungen erlauben.

Bei einer Kreiskeil-Verbindung sind, im Gegensatz zu einem zylindrischen Querpress-Verband, die Fügeflächen von Welle und Nabe nicht rund, sondern weisen am Umfang so genannte Keilflächen auf. Meist sind es drei Keilflächen. Sie bestehen aus identischen, gegeneinander verdrehten Abschnitten von Spiralen, z.B. logarithmischen Spiralen. Bei Verspannen durch Verdrehen um einen gewissen relativ kleinen Winkelbetrag (z.B. 15°) ergibt sich der notwendige homogene Flächenkontakt und damit der grösstmögliche Kraftschluss zwischen Welle und Nabe. Kreiskeil-Verbindungen gewährleisten einen günstigen Übergang der zu übertragenden Kräfte und besitzen eine vorteilhafte Gestaltfestigkeit. Eine Verbindung mit drei Kreiskeilen am Umfang ist selbstzentrierend. Wenn die rodiole Steigung der Keilflächen im Bereich von 1:50 bis 1:200 gewählt wird, ist eine solche Kreiskeil-Verbindung in der Regel selbsthemmend.

Bei sehr grossen Stückzahlen und nicht so hohen technischen Anforderungen kann das Kreiskeilprofil spanlos und daher relativ kostengünstig produziert werden. Andererseits erfordern kleinere Stückzahlen und hohe Qualitätsansprüche bislang eine fräs- oder sogar schleiftechnische Herstellung zu entsprechenden Kosten. Wegen des Durchmessers des Fräsers bzw. der Schleifscheibe entstehen dann an den Übergängen der einzelnen Kreiskeilflächen nicht nutzbare Bereiche. Diese führen zusammen mit dem zum Fügen erforderlichen relativen Verdrehwinkel zu einer nur partiellen kräftemässigen Ausnutzung der Verbindung.

Mit dem erfindungsgemässen Verfahren können derartige Kreiskeilverbindungen unter Anwendung verschachtelter Bearbeitungssequenzen nun mit hoher Genauigkeit und zu geringen Kosten selbst bei kleinsten Stückzahlen drehtechnisch hergestellt werden. Dabei wird sogar noch die Möglichkeit eröffnet, derartige Verbindungen im Bedarfsfall konisch auszulegen.

Die Erfindung soll im folgenden hinsichtlich der bevorzugten Anwendungen anhand der siebzehn Zeichnungsfiguren näher erläutert werden. Es zeigen:
- Fig.01: Hemisphärische Schraubpfanne mit kopfseitig klemmendem Flachgewinde gemäss dem Stand der Technik
- Fig.02: Hemisphärische Schraubpfanne mit einem mit Freiwinkel versehenen Flachgewinde gemäss dem Stand der Technik
- Fig.03: Erfindungsgemäss bearbeitete hemisphärische Schraubpfanne mit Flachgewinde aus Gewindeflügeln mit kopfseitigen Schraubflächen
- Fig.04: Erfindungsgemäss bearbeitete hemisphärische Schraubpfanne mit Spitzgewinde aus Gewindeflügeln mit allseitigen Schraubflächen
- Fig.05: Zwei Gewindeflügel der Schraubpfanne gemäss Fig.1
- Fig.06: Zwei Gewindeflügel der Schraubpfanne gemäss Fig.2
- Fig.07: Zwei Gewindeflügel mit Freiwinkel und bogenförmiger Kopffläche
- Fig.08: Zwei Gewindeflügel der Schraubpfanne gemäss Fig.3
- Fig.09: Zwei Gewindeflügel der Schraubpfanne gemäss Fig.4
- Fig.10: Drei Gewindeflüget der Schraubpfanne gemäss Fig.3 und hochdynamische Werkzeugbahn
- Fig.11: Drei Gewindeflügel der Schraubpfanne gemäss Fig.3 und Werkzeugbahn mittlerer Dynamik mit Sprungverfahren
- Fig.12: Drei Gewindeflügel der Schraubpfanne gemäss Fig.3 und überschwingende Werkzeugbahn mit Sprungverfahren
- Fig.13: Aus Sprungbefehlen erzeugte theoretische Werkzeugbahn
- Fig.14: Erzeugte Werkstückkontur aus Übergangsfunktionen
- Fig.15: Endgültige Werkstückgeometrie nach Weiterbearbeitung
- Fig.16: Hülse für eine Kreiskeilverbindung
- Fig.17: Zapfen für eine Kreiskeilverbindung

Die Zeichnungsfigur 1 stellt die polseitige Ansicht einer hemisphärischen Schraubpfanne 1 mit Flachgewinde gemäss dem Stand der Technik anhand eines Beispiels in einer etwa 1,3-fachen Vergrösserung dar. Für das Beispiel wurde der Nenndurchmesser auf 54 mm, die mittlere Zahnhöhe auf 2,6 mm, die Steigung auf 5 mm, und der Bodenlochdurchmesser auf 22 mm festgelegt. Diese Basisabmessungen wurden aus zeichnerischen Gründen gewählt und für die Zeichnungsfiguren 2 bis 4 zum Zwecke der besseren Vergleichbarkeit beibehalten. Ebenso wurde der Drallwinke) der Spannut einheitlich auf 0° gesetzt, um den zeichnerischen Aufwand zu reduzieren. Es ist bekannt, daß eine gedrallte Spannut Vorteile in Bezug auf einen günstigeren Spanwinkel und eine gleichmässiger verteilte Kraftübertragung mit sich bringt.

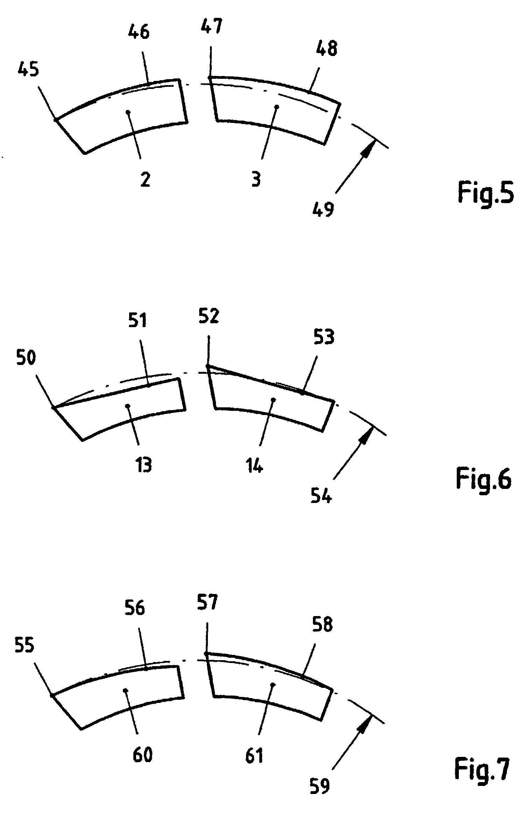

An das Bodenloch 9 der Schraubpfanne 1 schliesst sich ein kuppelförmiger gewindefreier Bereich 6 des Schalenkörpers an. Der Durchmesser des Schalenkörpers wird in der Zeichnung lediglich durch den äquatorialen Randbereich 10 repräsentiert. Der Gewindezug beginnt polseitig am ersten Gewindeflügel 7 und steigt bis vor den Gewindeflügel 2 auf seine volle Höhe an. Zwei der Gewindeflügel (2, 3) sind mit Kennziffern versehen, weil sie für eine Detaildarstellung in Fig.5 vorgesehen sind. Sowohl die kopfseitigen Flächen (4) als auch die jeweils am Zahnfuss zum Schalenkörper gebildeten Kanten (5) der einzelnen Gewindeflugel mit Ausnahme des Anfangs- bzw. Endbereichs des Gewindezugs - liegen in der zweidimensionalen Darstellung jeweils auf einer spiralförmigen Kurve. Dabei umfasst der gesamte Gewindezug etwa vier Umläufe. Der zwischen den Gewindeflügeln verlaufende Gewindegrund 8 bildet den hemisphärischen Mantel des Schalenkörpers. Zwecks Erzeugung von Spannuten (11) bzw. Schneidkonten ist die umlaufende Gewinderippe 12-fach ohne Drallwinkel geschlitzt. Dabei taucht die Schlitzung unter einem Winkel von etwa 10° ein, um am Gewindezahnkopf jeweils einen positiven Spanwinkel zu bilden.

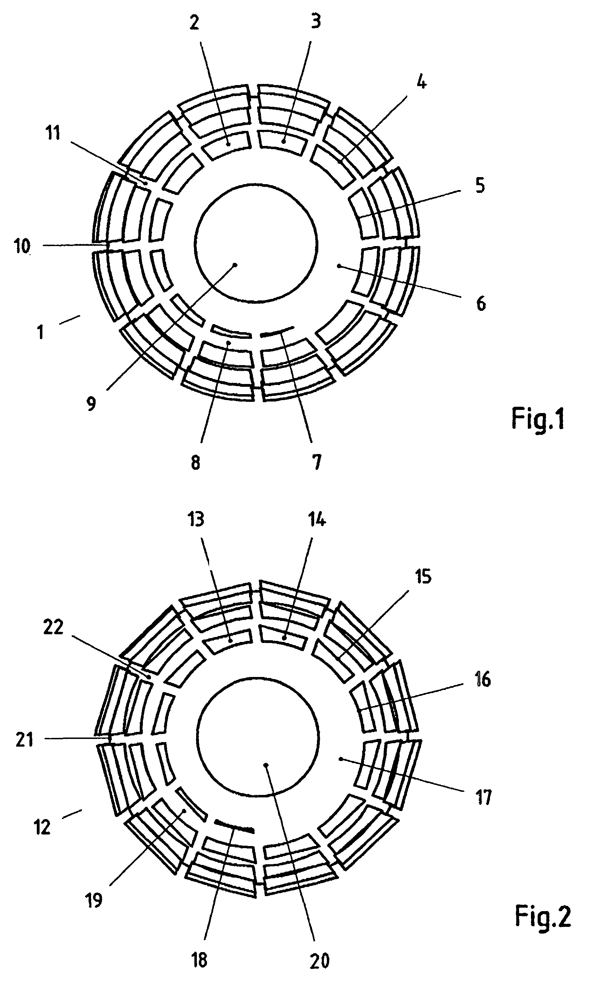

Das Ausführungsbeispiel einer Schraubpfonne 12 in Fig.2 mit Flachgewinde gemäss dem Stand der Technik ist durch eine frästechnische Nocharbeit aus der Schraubpfanne 1 hervorgegangen. Daher entsprechen Bodenloch 20, Kuppelbereich 17, Gewindegrund 19, Nenndurchmesser 21 und Schtitzung 22 ebenso wie die Konten (16) zwischen den Gewindeflügeln und dem Schalenkörper vollständig der Fig.1. Zwecks Aufrechterhaltung einer konstanten mittleren Gewindezahnhöhe wurden wegen der hemisphärischen Schalenkontur die Gewindeflügel einzeln nachgefräst. Dabei verschob sich der polseitige Gewindeanfang auf den Gewindeflügel 18. Die geraden Aussenflächen 15 der einzelnen Gewindeflügel verlaufen nun als Sehnen zum Schwenkkreis der in Einschraubrichtung jeweils vorn liegenden kopfseitigen Schneidkanten und in Synchronisation mit der Gewindeschlitzung so, dass in Bezug auf den jeweiligen Schwenkkreis Freiwinkel gebildet sind. Die Wirkung der Schneidkanten auf die Absenkung des Einschraubkraftbedarfs entfaltet sich durch den Umstand, dass der radiale Abstand der Schneidkanten von der Pfannenachse stets grösser ist als der entsprechende radiale Abstand des vorauslaufenden Flügelendes. Auf zwei der mit den Kennziffern 13 und 14 versehenen Gewindeflügel soll später in der Fig.6 noch näher eingegangen werden.

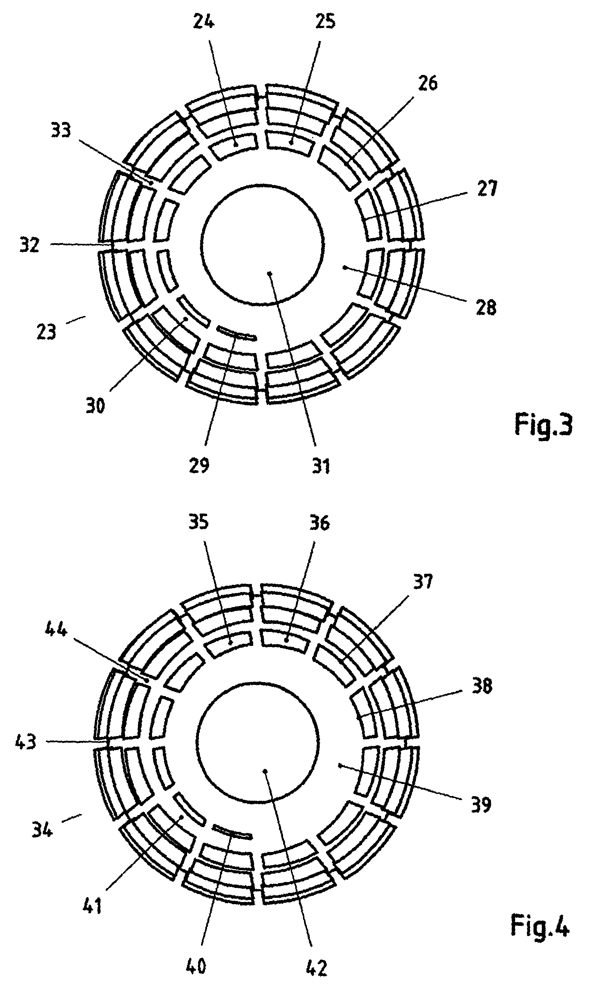

Das in Fig.3 gezeigte Ausführungsbeispiel einer mit dem erfindungsgemässen Verfahren bearbeiteten Schraubpfanne 23 entspricht in seiner hemisphärischen Scholenform und seinen Grundabmessungen, sowie dem Bodenloch 31, dem sich anschliessenden Kuppelbereich 28, der Kante (27) zwischen den Gewindeflügeln und dem Schalenmantel, dem Gewindegrund 30, dem Durchmesser 32 und der Gewindeschlitzung 33 wiederum dem Ausführungsbeispiel aus Fig.1. Der Gewindezug des Flachgewindes beginnt mit einem ersten Gewindeflügel 29 geringer Zahnhöhe, dem eine Abfolge von vier weiteren Gewindeflügeln mit jeweils sprunghaft sich vergrössernder Zahnhöhe folgen, bis die Gewinderippe mit dem Gewindeflügel 24 ihre volle Höhe erreicht. Die parallel verlaufenden Flanken jedes einzelnen Gewindeflügels grenzen jeweils an einen aussen liegenden Ausschnitt aus einer zur Schraubpfannenachse koaxialen Zylinderfläche 26, wobei der zugrunde liegende Zylinderdurchmesser von Gewindeflügel zu Gewindeflügel in Stufen zunimmt. Dieses Gestaltungsprinzip ist wahlweise auch mittels eines jeweiligen Ausschnitts aus einer entsprechend koaxial liegenden Schraubfläche realisierbar. Durch die beschriebene Gestaltung ist an den Gewindeflügeln weder ein Klemm- noch ein Freiwinkel gebildet. Ein Freiwinkel ist dort überhaupt nicht erforderlich, weil die von der (z.B. durch Sandstrahlen der Schraubpfannenoberfläche erzeugten) Oberflächenrauhigkeit ausgehenden Raspelkräfte bei neutraler Relativbewegung ein Verklemmen während des Einschraubvorgangs verhindern. Damit ist zunächst die nachteilige Spaltenbildung zwischen dem Implantat und dem knöchernen Lager unterbunden. Trotzdem kommt die jeweils vorn aussen liegende Schneidkante der Gewindeflügel zur Geltung, weil sie einen größeren radialen Abstand von der Pfannenachse hat als die voranlaufende Schneidkante. Das Ergebnis ist ein etwas niedrigerer Einschraubkraftbedarf bei mittlerer Taktilianz, sowie eine verbesserte Primärund Sekundärfixation des Implantats.

Ein weiteres Ausführungsbeispiel einer mit dem erfindungsgemässen Verfohren bearbeiteten hemisphärische Schraubpfanne 34 wird in Fig.4 vorgestellt. Auch hier wurden verschiedene Einzelheiten, namentlich das Bodenloch 42, der Kuppelbereich 39, der Gewindegrund 41, der Durchmesser 43 und die Gewindeschlitzung 44 von den zuvor gezeigten Ausführungsbeispielen unverändert übernommen. Im Gegensatz dazu handelt es sich bei dem dargestellten Gewinde um ein Spitzgewinde mit im Prinzip dreieckigem Gewindezahnprofil. Diese Tatsache ist der zweidimensionalen Darstellung nicht zu entnehmen. Ähnlich wie zuvor beginnt der Gewindezug mit einem ersten kleinen Gewindeflügel 40 und steigt in seiner Zahnhöhe in mehreren Stufen an, um vor dem Gewindeflügel 35 seine endgültige (mittlere) Zahnhöhe zu erreichen. Die vom Zahnkopf gebildete Kante (37), welche bei einem wirklich spitzen Dreiecksquerschnitt des Gewindezahns praktisch nur als Linie existiert, ist für jeden einzelnen Gewindeflügel eine Schraubenlinie mit konstantem Abstand von der Schraubpfannenachse, welche der Zeichnung nur als Bogen mit einem vom Pfannenmittetpunkt ausgehenden festen Radius zu entnehmen ist. Bei dem gewählten Spitzgewinde ist wegen des fehlenden Dralls der Spannut 44 eine Schneidkante an beiden Gewindezahnflanken gebildet. Die Schneidkante verlagert sich auf eine der Gewindezahnflonken, wenn ein entsprechender Drollwinkel der Spannut existiert. Die beidseitigen Flächen eines einzelnen Gewindeflügels des gezeigten Beispiels sind Schraubflächen, wobei die Steigung der polseitigen Fläche der Steigung der äquatorseltigen Fläche entspricht, auch wenn der optische Eindruck wegen des zum Äquator hin zunehmenden Pfannendurchmessers eine andere Situation vortäuscht. Dadurch scheint die am Zahnfuß zwischen Gewindeflügel und Schalenmantel der Schraubpfanne gebildete Kante 38 nach rückwärts in den Schalenmantel hinein zu verfaufen. Nachdem für die Schraubflächen des beim Einschrauben jeweils nächstfolgenden Gewindeflügels grössere radiale Abstände von der Pfannenachse herangezogen werden, stehen die beidseitigen Schneidkonten gegenüber dem jeweils vorauslaufenden Gewindeflügel seitlich zum Gewindeprofil bzw. radial nach aussen vor und sorgen so für einen leichtgängigen Schnitt beim Einschrauben. Auch in diesem Fall ist wegen des von den Gewindeflugeln in ihrer Erstreckung gebildeten Neutralwinkels das Auftreten von Spalten im Kontaktbereich zum Knochen unterbunden.

Die oben zum Stand der Technik und zu Ausführungsbeispielen des erfindungsgemässen Verfahrens gemachten Aussogen sollen im folgenden anhand vergrößert herousgezeichneter Einzelheiten besser verdeutlicht werden, weil bestimmte Details in der jeweiligen Gesamtansicht nur schwer zu erkennen sind.

In Fig.5 sind zwei Gewindeflügel 2, 3 der Fig. 1 vergrössert herausgezeichnet. Davon besitzt der Gewindeflügel 2 eine an der Stirn seiner kopfseitigen Fläche 46 liegende Schneidkante 45, und der Gewindeflügel 3 eine gleiche Schneidkante 47 an der entsprechenden Fläche 48. Der von der Schneidkante 45 während des Einschraubens der Schraubpfanne beschriebene Schwenkkreis 49 mit einem festen Radius um die Pfannenmittelachse ist als strich-punktierte Linie eingetragen. Es ist gut zu erkennen, dass ein Teil des jeweiligen Gewindeflügels über den Schwenkkreis herauswächst, was generell zu Klemmeffekten führen muss.

Bei der in Fig.6 gezeigten Ausführung der Gewindeflügel 13, 14 gemäss dem Beispiel aus Fig.2 sind solche Klemmeffekte nicht zu befürchten, da die kopfseitigen Flächen 51 bzw. 53 nach den Schneidkanten 50 bzw. 52 mit einem Freiwinkel hinterfräst sind. Dabei wird der strich-punktierte Schwenkkreis 54 der Schneidkante 50 an keiner Stelle von der kopfseitigen Fläche des Gewindeflügels berührt. Allerdings verbleibt in diesem Bereich jeweils ein unerwünschter Freiraum. Dieser ist um so grösser, je kleiner die Zahl der Spannuten ist. Hier sind insbesonders Schraubpfdnnen mit z.B. nur sechs Spannuten in extremer Weise gehandicapt. Die gezeigte Gestaltung wird gerne bei konischen Schraubpfannen benutzt, weil dann die Gewindeflüget sozusagen im Paket sehr rationell überfräst werden können. Aus medizinischer Sicht ist dieses Argument jedoch abzulehnen.

Der oben aufgezeigte Problempunkt ist mittels einer Gestaltung der Gewindeflügel 60, 61 gemäss Fig.7 in gewissem Umfang abschwächbar. Auch hier sind die kopfseitigen Flächen 56, 58 der Gewindefiügel hinter den stirnseitigen Schneidkanten 55 und 57 mit einem Freiwinkel bezüglich des Schwenkkreises 59 versehen, sodaß ein Verklemmen beim Einschrauben verhindert wird. Wegen der Bogenform der Flächen 56, 56 ist jedoch der spaltbildende Freiraum relativ klein und daher eher akzeptabel. Allerdings erforderte diese Bogenform bisher einen hohen frästechnischen Aufwand, weil die einzelnen Gewindeflügel bei der Herstellung im Prinzip einzeln tangential abgefahren werden mußten. Mit dem erfindungsgemässen Verfahren kann die gezeigte geometrische Gestaltung der einzelnen Gewindeflögel jetzt sehr rationell in nur einer Aufspannung auf einer CNC-Drehmaschine hergestellt werden.

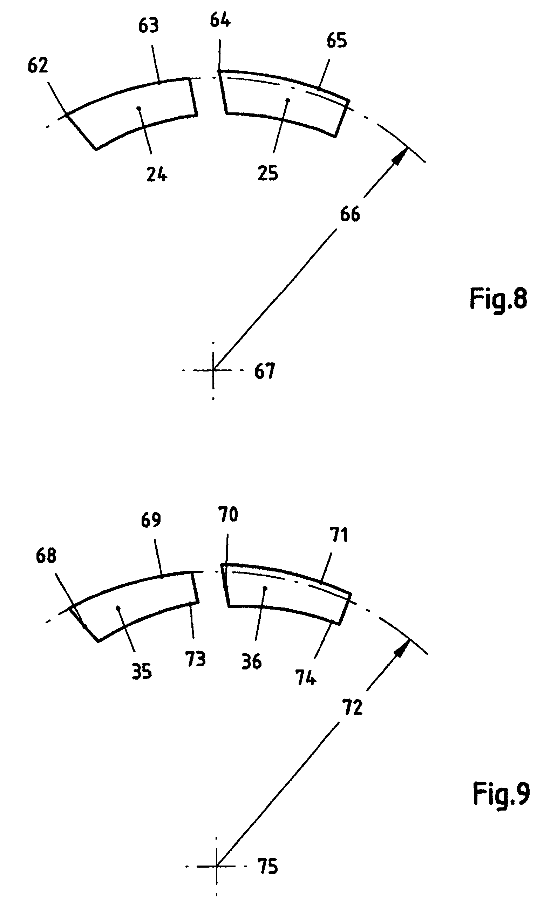

Zum Vergleich wird die mit dem erfindungsgemässen Verfahren herstellbare Ausführung der jeweiligen Aussenflächen der einzelnen Gewindeflügel als sogenannte Schraubflächen, wie sie bereits in Fig.3 vorgestellt wurde, in Fig.8 anhand von zwei vergrössert abgebildeten Gewindeflügeln 24, 25 gezeigt. Die jeweils von den Schneidkanten 62 bzw. 64 ausgehenden Kopfflächen 63 bzw. 65 der Gewindeflügel besitzen einen festen Radius, welcher jeweils ab Abstand der Schneidkante von der Schraubpfannenachse 67 definiert ist. In der Zeichnung fällt daher der durch die Schneidkante 62 verlaufende, strich-punktiert dargestellte Schwenkkreis aus dem festen Radius 66 deckungsgleich mit der Kopffläche 63 zusammen. Da der entsprechende Radius des Gewindeflügefs 25 grösser ist, rogt dessen Schneidkante 64 gegenüber der beim Einschrauben vorauslaufenden Schneidkante 62 des Gewindeflügeis 24 vor. So kann die jeweilige Schneidkante und die sich anschliessende, in einem positiven Spanwinkel angestellte Stirnfläche in das zu zerspanende Knochen material eindringen und mit relativ leichtem Schnitt die Späne in die Spannut hinein abführen.

Die in Fig.9 vergrössert aus der Fig.4 herausgezeichnete Situation unterscheidet sich gegenüber der Ausführung in Fig.8 dadurch, dass das Gewinde in seinem Zahnprofil nun nicht ein Flach- sondern ein Spitzgewinde ist. Jedoch sind auch hier alle äusseren Flächen der einzelnen Gewindeflügel 35, 36 jeweils als Schraubflächen gestaltet. Wegen des schrägen Seitenwinkels und der Steigung bzw. Anstellung der Gewindeflügel, sowie der hemisphärischen Schalenkontur scheint die jeweils am Zahnfuss zum Schalenmantel gebildete Kante mit ihrem rückwärtigen Ende 73, 74 in den Schalenkörper hinein zu verlaufen. Tatsächlich tritt jedoch bei der Rotation der Schraubpfanne kein radiales Schieben des projizierten Zahnquerschnitts auf, weil die jeweiligen Außenkanten 69, 71 in ihrem Radius zur Schraubpfannenachse unveränderlich sind. Aus der Heranziehung eines dreieckigen Zahnquerschnitts für das gezeigte Beispiel ergibt sich eine Verlagerung der jeweiligen Schneidkante auf mindestens eine, bzw. für Spannuten ohne Drall, auf beide Seitenflächen des jeweiligen Gewindeflügels. In der Zeichnung ist nur die jeweils polseitige Schneidkonte 68, 70 zu sehen. Die jeweils rückwärtige Schneidkante ist verdeckt. Der Schwenkkreis der kopfseitigen Gewindezahnkante 69 ist mit dem festen Radius 72 um die Schraubpfannenachse 75 dargestellt. Der extrem reduzierte Einschraubkraftbedarf dieser Ausführung ergibt sich aus dem gegenseitigen radialen Versatz der einzelnen Gewindeflügel, wodurch die einzelnen Schneidkonten gegenüber den jeweils vorauslaufenden sowohl seitlich als auch nach aussen vorstehen.

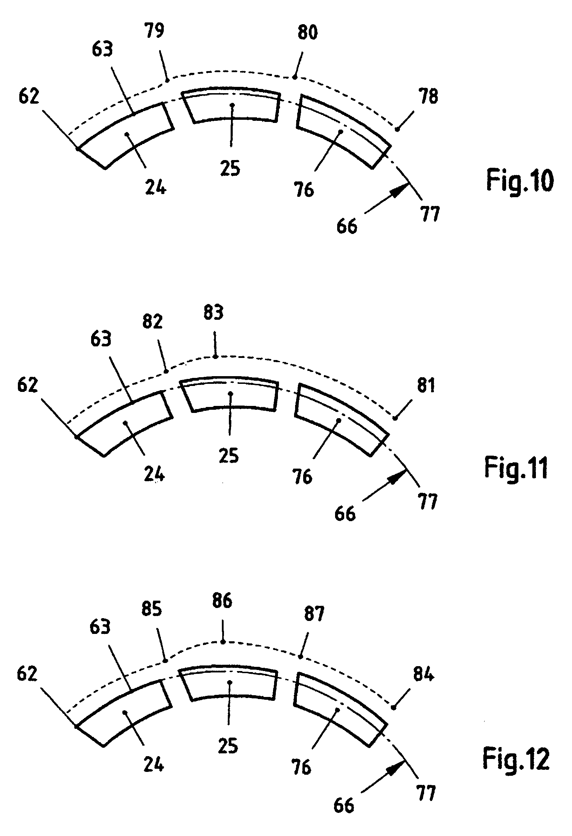

Zum besseren Verständnis der Vorgehensweise zur Ausführung des Verfahrens für die vorgeschlagene bevorzugte Anwendung zur Herstellung eines Schraubpfannengewindes werden die aus Fig.3 und 8 bekannten Gegebenheiten in den Figuren 10 bis 12 nochmals aufgegriffen. Es werden hier in jeder der Figuren die drei Gewindeflügel 24, 25, 76 des Flachgewindes abgebildet, sowie die Schneidkante 62 an der kopfseitigen Fläche 63 und deren strich-punktierter Schwenkkreis 77 mit dem von der Schraubpfannenachse ausgehenden Radius 66. Dabei wurde der Abbildungsmassstab gegenüber den vorangegangenen Figuren geringfügig verkleinert.

In der Fig.10 ist eine von einem Bearbeitungswerkzeug (z.B. Wendeschneidplatte) beschriebene, äquidistant zu den kopfseitigen Flächen der einzelnen Gewindeflügel versetzte Bahn 78 dargestellt, welche in der gezeigten Gestalt mittels einer entsprechenden Programmierung mit einer extrem dynamischen Drehmaschine erzielbar ist. Der Abstand der Bahn von der zu zerspanenden Kontur wurde deshalb gewählt, um den Bahnverlauf in seiner vollständigen Erstreckung sichtbar zu machen. In der Bahn 78 sind zwei Unstetigkeiten 79 und 80 enthalten, welche über die Programmierung bewusst auf eine Position verlegt wurden, welche bei der nachfolgenden Bearbeitung zur Gewindeschlitzung durch Fräsen entfernt wird. Obwohl die Unstetigkeiten 79, 80 der Bahn 78 Übergangsfunktionen sind, wird so zwischen den aufeinander folgenden Gewindeflügeln eine radiale Sprungfunktion bewirkt. Diese radiale Sprungfunktion besteht auf jeden Fall bezüglich der vorgeschlagenen Programmierung, wobei mindestens zwei aufeinander folgende Koordinaten gleichen Durchmessers mit einem der Bearbeitungsaufgabe angepaßten Verfahrweg in Z sowie eine entsprechende Steigung bzw. die entsprechenden Spindelwinkel, und im Anschluss ein Durchmessersprung mit maximalem Vorschub (z.B. 100 mm/U) eingegeben werden müssen. Für ein akzeptables Bearbeitungsergebnis ist es erforderlich, dass der Übergangsbereich am Werkstück nicht breiter ist, als die vorgesehene Breite der Spannut.

Mit den meisten der heute verfügbaren CNC-Drehmaschinen ist die Erzeugung der in Fig.10 gezeigten Schneidenbahn nicht möglich, weil ihre Gesamtdynamik nicht ausreicht, den Kreuzschlitten innerhalb der geforderten Strecke auf einen anderen Drehdurchmesser zu bewegen und dabei gleichzeitig eine ausreichende Bahngenauigkeit einzuhalten. Mit der Erfindung wird für diese Fälle ein Sprungverfahren vorgeschlagen, mit welchem dieses Problem prinzipell überwindbar ist. Der entsprechende theoretische Hintergrund soll mittels der Fig.11 verdeutlicht werden. Die anhand der Bahnkurve 81 dokumentierte Arbeitsweise sieht vor, mit einer ersten Bearbeitungssequenz lediglich den z.B. ersten, dritten, fünften, siebten usw. Gewindeflügel zu bearbeiten und dann den zweiten, vierten, sechsten usw. auszulassen. Die sich aus der Programmierung mit Sprungfunktionen aufgrund der Maschinendämpfung jeweils ergebende Obergangsfunktion der Bahn 81 muß dabei lediglich ausreichen, nach dem Punkt 82 der ersten Reaktion das Werkzeug so über die nächstfolgende Schneidkante zu heben, dass diese nicht verrundet oder beschädigt wird. Für die Rückführung des Werkzeugs auf die angestrebte Bahn steht dann z.B. bis zum Punkt 83 eine Strecke zur Verfügung, die nicht durch die Spannutenbreite limitiert ist. Es ist dann ohne weiteres möglich, in einer zweiten Arbeitssequenz die ausgelassenen Konturelemente nachzuholen und dabei die bereits bearbeiteten entsprechend zu überspringen.

Bei älteren Drehmaschinen mit entsprechender Trägheit des Regelkreises muss damit gerechnet werden, daß ein Überschwingen die Bahnkurve zusätzlich verzerrt. Dieser Effekt soll mittels der Bahn 84 in Fig. 12 verdeutlicht werden. Nach dem abrupten Reagieren der Werkzeugbewegung auf die programmierte Vorgabe bei Punkt 85 tritt ein Überschwingen der Bahn auf, welches bei Punkt 86 sein Maximum erreicht. Dieses wird im Anschluss weich auslaufend abgebaut, bis die Bahn etwa bei Punkt 87 wieder der programmierten Vorgabe entspricht. In dem Beispiel wäre der beschriebene Effekt mittels des vorgeschlagenen Sprungverfahrens in zwei Bearbeitungssequenzen gerade noch beherrschbar. Im gegebenen Fall könnte das Sprungverfahren jedoch ohne weiteres auf drei oder mehr Sequenzen ausgedehnt werden.

Das oben in verschiedenen Varianten erläuterte Verfahren ist für geschrägte Zahnkopfflächen ebenso anwendbar wie für die Seitenflächen von Gewindeflügeln z.B. gemäss Fig.9. Dabei verlagern sich die beschriebenen Sprungfunktionen entweder ganz oder teilweise von der X- auf die Z-Achse. Für diese Fälle werden die vom Werkzeug beschriebenen Humpelbahnen hier zeichnerisch zwar nicht dargestellt, entsprechen jedoch vom Prinzip her denjenigen des gezeigten Sprungverfahrens für die Zahnkopfbearbeitung.

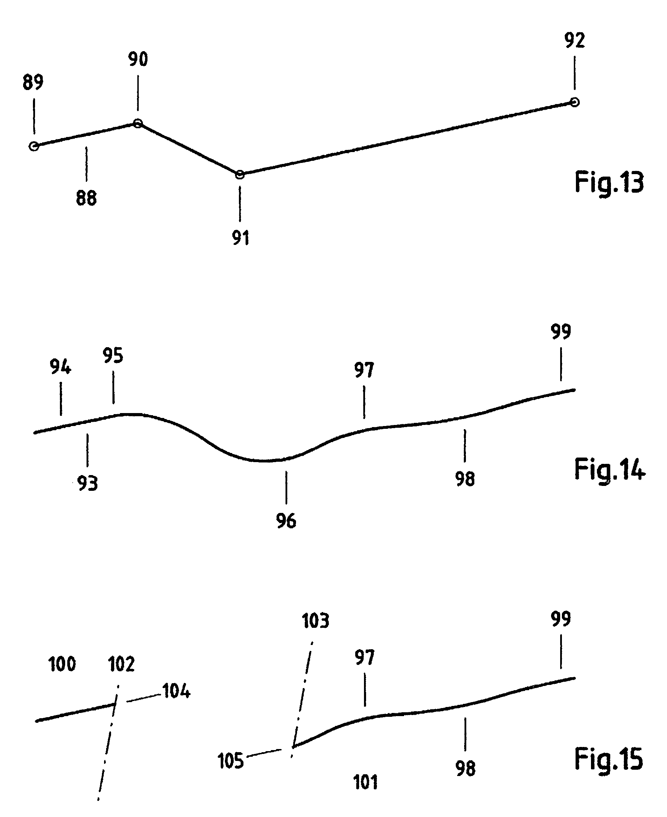

Wie bereits weiter oben beschrieben wurde, eröffnet die Erfindung auch die Möglichkeit, das Überschwingverhalten der Maschine direkt zur Erzeugung von Freiwinkeln an Gewindeflügeln nutzbar zu machen. Mittels der Figuren 13 bis 15 soll die genaue Vorgehensweise näher erläutert werden. Die Figuren 13 bis 15 zeigen anhand eines auf eine versetzte Zahnflanke bezogenen schematischen Beispiels drei überhöht dargestellte Kurven, welche unter Weglassung der räumlichen Komponente auf die Verdeutlichung des interessierenden Bewegungsanteils der Werkzeugbahn reduziert wurden. In der Praxis kann dieser Bewegungsanteil in einer oder in mehreren Ebenen liegen.

In Fig. 13 ist die mittels der Programmierung befohlene Werkzeugbahn 88 mit einem einzigen Sprungbefehl dargestellt. Die Koordinatenpunkte 89, 90, 91 und 92 sind mittels entsprechender Werte für X und Z angegeben. Davon ist auf dem Zeichnungsblatt lediglich die Änderung von Z als vertikale Komponente sichtbar, während die jeweilige Grösse von X aus der Zeichnung nicht entnehmbar ist. Die horizontalen Abstände zwischen den Koordinatenpunkten sind proportional dem jeweiligen Spindelwinkel, welcher entweder direkt über den Parameter C (Spindelwinkel) oder indirekt über F (Steigung) programmierbar ist. Dabei ist zu beachten, dass bei Heranziehung des Parameters F der für die jeweilige MC-Steuerung zulässige Grösstwert nicht überschritten wird, während für die Spindelwinkelprogrammierung der Winkelsprung ohne weiteres 0° betragen kann. Prinzipiell sind auch mehrere Sprungbefehle miteinander verkettbar.

Fig.14 zeigt den an einem Werkstück vor der Ausfräsung der Spannut gemessenen Verlauf einer Gewindezahnflanke, welcher aus der Befehlskette gemäß Fig. 13 resultiert. Die abgebildete Kurve 93 besteht aus Übergangsfunktionen, welche in der Trägheit und Regelsteifigkeit der Maschine und der Steuerung begründet sind. Sie beginnt mit einem glatten Verlauf 94, um bei Punkt 95 synchron dem Sprungbefehl abrupt auszulenken. Dabei wird ein Punkt maximaler Überschwingung 96 erreicht, dem sich ein Rückschwung 97 anschliesst. Danach folgt ein Nachschwingen 98 kleinerer Amplitude, bevor die Kurve wieder in einen stetigen Verlauf 99 übergeht.

In Fig.15 wird die seitliche Werkstückkontur nach der Herstellung der Sponnut gezeigt. Die Flanken der Spannut sind durch zwei strich-punktierte Linien 102, 103 angedeutet. Es sind nun die Flanken 100, 101 von zwei Gewindeflügeln gebildet. Die Lage der Spannut ist mit der Kontur der Gewindezahnflanke derart synchronisiert, dass einerseits dos Ende 104 des vorlaufenden Gewindeflügels vor die Auslenkung bei Punkt 95 gelegt, und andererseits ein mit einem Freiwinkel versehener Überstand der Schneidkante 105 am nachfolgenden Gewindeflügel gebildet ist. Der durch das Nachschwingen verursachte kleine Buckel 98 ist in seiner Amplitude abhängig sowohl von der zum Einsatz kommenden Maschine und Steuerung, als auch z.B. von der angewandten Schnittgeschwindigkeit. Für die generelle Wirksamkeit der hauptsächlich erzeugten vorstehenden Schneidkante und deren Freiwinkel ist er praktisch ohne Bedeutung.

Der in der Zeichnungsfigur beispielhaft dargestellte Verlauf von zwei aufeinander folgenden Gewindezahnflanken bezieht eine gegenseitige Verschwenkung der einzelnen Gewindeflügel in ihrer Erstreckungsrichtung ein. Der Grad dieser Verschwenkung hängt von den konstruktiven Vorgaben ab. Die Verschwenkung kann dabei derart minimiert oder gänzlich aufgehoben werden, dass lediglich ein Relikt der Überschwingung (96) in Gestalt der Schneidkante 105, bzw. einem Teil von ihr, über das Ende 104 des vorauslaufenden Gewindeflügels vorsteht.

Das mit Hilfe der Zeichnungsfiguren 13 bis 15 erläuterte Verfahren ist in einer entsprechenden Weise z.B. bei Flachgewinden am radial nach aussen zeigenden Zahnkopf anwendbar, sowie bei anderen Gewinden an zwei oder mehreren Flächen des Gewindezahnprofils.

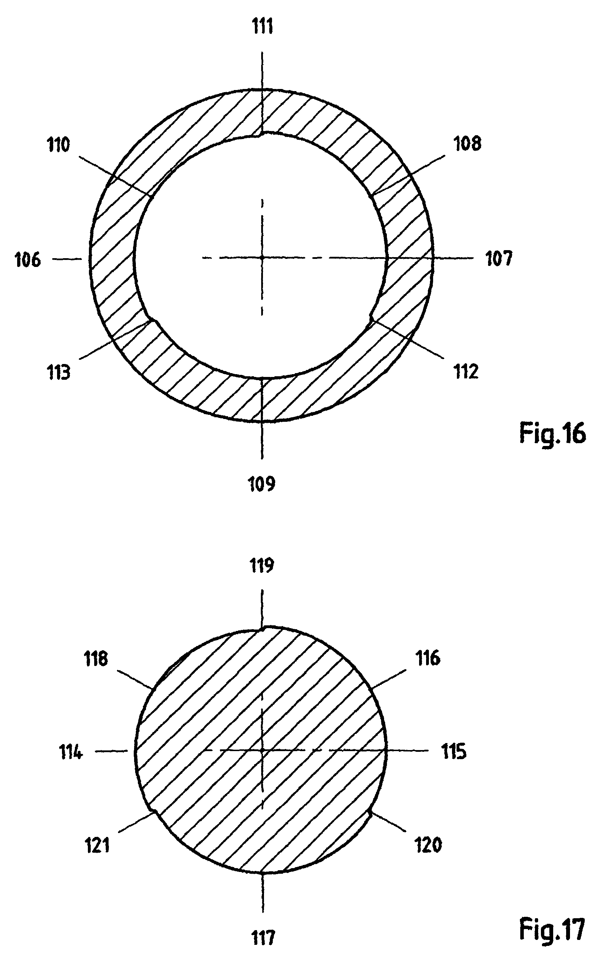

Eine weitere Anwendung des erfindungsgemässen Verfahrens soll mittels der Figuren 16 und 17 an Hand eines Beispiels vorgestellt werden. Dabei handelt es sich um eine so genannte Kreiskeil-Verbindung, welche im allgemeinen Maschinenbau Verwendung findet. Die Fig.16 zeigt eine Kupplungshülse 106 mit ihrem Zentrum 107. An der inneren Wandung sind drei Kreiskeilflächen 108, 109, 110 gebildet, welche mit Sprüngen 111, 112, 113 aneinander stossen. Ein dem inneren Profil der Hülse 106 angepasster Zapfen 114 ist in Fig. 17 dargestellt. Dieser weist drei zur Mittelachse 115 zentrierte äussere Kreiskeilflächen 116, 117, 118 auf, welche mit Sprüngen 119, 120, 121 ineinander übergehen. Die sowohl an Hülse 106 und Zapfen 114 vorhandenen Kreiskeifflächen sind Abschnitte aus Spiralen, welche an den jeweiligen Stossstellen abrupt beginnen bzw. enden. Für die Herstellung dieser Kreiskeilflächen mittels des erfindungsgemässen Verfuhrens ist es im Prinzip gleichgültig, ob es sich dabei um Abschnitte aus einer archimedischen, logarithmischen, hyperbolischen oder fermatischen Spirale hondelt. Man geht allerdings davon aus, dass eine Kreiskeilfläche aus einer logarithmischen Spirale wegen des gleichbleibenden Steigungswinkels die günstigste Materialbeanspruchung beim Verspannen erzeugt.

Bei der Herstellung der inneren bzw. äusseren Kreiskeilflächen kommt es darauf an, einen der Vorgabe weitestgehend entsprechenden Krümmungsverlauf zu realisieren und dabei an den Sprüngen möglichst wenig von der späteren Kontaktfläche zu verschenken. Diese Aufgabe ist mittels des erfindungsgemässen Verfahrens unter Enbeziehung des weiter oben schon beschriebenen Sprungsystems ohne weiteres lösbar. Zur spanenden Herstellung z.B. der Kreiskeilhülse 106 auf einer CNC-Drehmaschine wird der entsprechende Rohling zunächst vorgebohrt und gegebenenfalls mittels eines Schruppgangs auf das Vormass bearbeitet. Die Fertigbearbeitung mittels einer Bohrstange z.B. mit Wendeschneidplatte erfolgt im Prinzip derart, dass während der Werkstückrotation das Werkzeug mit einem kleinen Vorschub bis zum Ende der Kreiskeilfläche radial nach aussen verfahren und dann mittels eines nach innen gerichteten Sprungbefehls von der Kreiskeilfläche abgehoben wird. Aus diesem Sprungbefehl im Programm entsteht eine aus Übergangselementen bestehende Werkzeugbahn mit einem zum Zentrum 107 zeigenden Überschwung, welcher mittels der Programmierung derart dimensioniert ist, dass das Werkzeug vom Beginn der nächsten Kreiskeilfläche deutlich entfernt ist. Die im Programm folgenden Befehlssätze sind darauf ausgelegt, die nächste Kreiskeilfläche zu überspringen und das Werkzeug auf einer beruhigten Bahn in die übernächste Kreiskeilfläche eintreten zu lassen. Für das in Fig.16 gezeigte Ausführungsbeispiel, welches eine in der Aufsicht rechtsdrehende Relativbewegung des Werkstücks zum Drehmeissel erfordert, wird dann die Bearbeitungsabfolge der drei Kreiskeilflächen 108, 109, 110, z.B. beginnend an der Kreiskeilfläche 108 derart lauten:
108 - bearbeiten von 112 bis 111
110 - überspringen
109 - bearbeiten von 113 bis 112
108- überspringen
110 - bearbeitet von 111 bis 113
109 - überspringen
108 - bearbeiten von 112 bis 111
usw.

Für die erfindungsgemässe Auslegung des NC-Programms bestehen eine Reihe von Freiheiten. So kann beispielsweise der radiale Vorschub als Steigung wahlweise mit einer überlagerten Änderungsfunktion (z.B. mittels des Parameters E) oder über feste Koordinaten programmiert werden, um eine bestimmte Art der Röchenkrümmung zu verwirklichen. Hinsichtlich der axialen Werkzeugbewegung besteht die Wohl darin, den entsprechenden Werkzeugvorschub entweder beizubehalten und einen kleineren Vorschubwert zu benutzen, oder einen Vorschub nur entweder während der Zerspanung der einzelnen Kreiskeilflächen oder den Zerspanungspausen beim Überspringen anzuwenden.

Im übrigen entspricht die Herstellung der Kreiskeilflächen des passenden Zapfens im Prinzip der für die Hülse beschriebenen Vorgehensweise. Es ist eine entsprechende Tolerierung der Abmessungen zu berücksichtigen, so dass beide Teile zusammenfügbar sind. Die aus der erfindungsgemässen Beorbeitung sich ergebenden Sprungflächen machen einen derart kleinen Teil des Umfangs aus, dass zwischen den gefügten Partnern nur kleinste von der Kraftübertragung ausgeschlossene Lücken gebildet sind.

Tatsächlich sind die mit dem Verfahren gebotenen Möglichkeiten nahezu unbegrenzt. Sie ergeben sich aus der Anwendung von CNC-Programmen unter Verknüpfung der Schlittenbewegung mit der Spindeldrehung der Maschine und der Einbeziehung bzw. der Kombination von Humpeiwerten der Adressparameter für Durchmesser, Länge, bzw. Steigung oder Spindelwinkel, sowie wahlweise der Benutzung einer Pilgerschritt-Technik bzw. der beschriebenen schachtelbaren Bearbeitungssequenzen. Damit sind jetzt Bearbeitungen auf CNC-Drehmaschinen sehr rationell möglich, welche zuvor zeitaufwendig und teilweise in schlechterer Oberflächenqualität durch Fräsen erzeugt werden mussten.

Die für die Anwendung des Verfahrens vorgeschlagene künstliche Hüftgelenkpfanne mit speziellem Gewinde und Gewindeflügeln aus Schraubflächen mit Neutralwinkeln hinter den Schneidkanten überzeugt durch sehr niedrige Einschraubkräfte, eine extreme Sicherheit gegen Überdrehung, eine hervorragende Taktilianz und durch weitestgehend spaltenfreie Übergänge zur knöchernen Lagerfläche. Besonders vorteilhaft ist eine solche Ausführung mit Spitzgewinde, gedrallten Spannuten und relativ zueinander in Richtung zum Drallwinkel verschwenkten Gewindeflügeln. Damit ist nicht nur die Handhabung während der Implantation deutlich verbessert, sondern auch die Primär- bzw. Sekundärfixation erheblich gesteigert und damit die Gefahr vorzeitiger Auslockerung nahezu ausgeschlossen.

## Patentansprüche

1. Drehtechnisches Verfahren zum Unrunddrehen auf einer programmierbaren Drehmaschine, wobei ein Werkstück im Futter einer Maschinenspindel rotiert und dabei mit einem Werkzeug am Werkstück mindestens partiell bestimmte unrunde, z.B. aus geometrischen Übergangselementen gebildete oder zusammengesetzte Konturen spanend erzeugt werden, **dadurch gekennzeichnet, dass** das Drehen humpelnd erfolgt, indem der Kreuzschlitten mit dem Zerspanungswerkzeug synchronisiert zum Spindetwinkel verfahren wird und die unrunden Konturen mittels einer Programmierung aus Sprungfunktionen durch Verknüpfung von Befehlssätzen mit Werten für ausgewählte Adressparameter wie z.B. Durchmesser (X), Länge (Z), Steigung (F) oder C (Winkel) erzeugt werden, wobei mindestens für einen dieser Adressparameter in der Programmsatzkette eine humpelnde, d.h. eine Sprungfunktion aufweisende Folge aus Adressparameterwerten verwendet wird.

2. Verfahren gemäss Anspruch 1, **dadurch gekennzeichnet, dass** in den Befehlssätzen zusätzlich der Parameter Höhe (Y) benutzt wird.

3. Verfahren gemäss einem der Ansprüche 1 oder 2, **dadurch gekennzeichnet, dass** eine Gewindeprogrammierung benutzt wird.

4. Verfahren gemäss einem der Ansprüche 1, 2 oder 3, **dadurch gekennzeichnet, dass** für mindestens zwei der genannten Adressparameter in der Programmsatzkefte eine humpelnde Folge aus Adressparameterwerten verwendet wird.

5. Verfahren gemäss einem der vorgenannten Ansprüche, **dadurch gekennzeichnet, dass** die Programmsatzkette eine rotationssymmetrische Kontur mit einer überlagerten nicht monotonen periodischen Folge von Inkrementen beschreibt.

6. Verfahren gemäss einem der vorgenannten Ansprüche, **dadurch gekennzeichnet, dass** für mindestens einen der Adressparameter die zwischen den Adressparameterwerten der Programmsatzkette gebildeten Inkremente als humpelnde Folge programmiert sind.

7. Verfahren gemäss einem oder mehreren der vorgenannten Ansprüche, **dadurch gekennzeichnet, dass** die Unstetkontur durch die Programmierung eines Pilgerschrittverfahrens erzeugt wird, indem das Werkzeug mit einer Abfolge von Vorwärts- und Rückwärtsbewegungen verfahren wird, wobei eine der Bewegungen grösser ist als die andere.

8. Verfahren gemäss einem oder mehreren der vorgenannten Ansprüche, **dadurch gekennzeichnet, dass** die Unrund- bzw. Unstetkontur am Werkstück durch Verschachtelung von mindestens zwei Bearbeitungssequenzen erzielt wird, wobei mittels einer ersten Sequenz ein erstes Konturelement erzeugt, das nächste Konturelement übersprungen, das darauf folgende Konturelement wiederum erzeugt wird, und mit einer zweiten Sequenz das oder die übersprungenen Konturelemente bearbeitet und dabei die bereits bearbeiteten Konturelemente übersprungen werden.

9. Verfahren gemäss einem oder mehreren der vorstehenden Ansprüche für die spanende Erzeugung unstet verlaufender, aus einer geneigten oder gekrümmten Mantelfläche herausstehender Konturelemente, wobei mit der Seite des Drehmeissels im wesentlichen die Flanke des unstet verlaufenden Konturelements und mit der Spitze des Drehmeissels im wesentlichen die Mantelfläche bearbeitet wird, **dadurch gekennzeichnet, dass** die Spitze des Drehmeissels auf einer im wesentlichen tongential zur Mantelfläche verlaufenden Bahn geführt wird und die Seite des Drehmeisseis mittels einer programmierten Änderung der tangentialen Verfahrgeschwindigkeit und/oder Verfahrrichtung die Flanke des unstet verlaufenden Konturelements erzeugt.

10. Verfahren gemäss einem oder mehreren der vorgenannten Ansprüche, **dadurch gekennzeichnet, dass** das aus einem Sprungbefehl der Programmierung resultierende Überschwingverhalten der Drehmaschine direkt zur Erzeugung unsteter, unrunder bzw. gekrümmter Konturen herangezogen wird.

11. Verfahren gemäss Anspruch 10, **dadurch gekennzeichnet, dass** das Überschwingverhalten der Drehmaschine zur direkten Erzeugung von Schneidkanten mit Freiwinkeln an Gewindesegmenten bzw. -flügeln verwendet wird.

12. Verfohren gemäss Anspruch 11, **dadurch gekennzeichnet, dass** die Schneidkonten durch wenigstens partielles Herausfräsen von Sponnuten im Bereich von aus einem Überschwingverhalten der Drehmaschine hervorgegangenen Abschnitten der Gewindeflügel erzeugt werden und dabei die Freiwinkel Relikte des Überschwungs darstellen.

13. Anwendung des Verfahrens gemäss einem oder mehreren der Ansprüche 1 bis 12 für die spanende Herstellung von speziellen Gewinden an Einschraubkörpern, z.B. für nachgiebige Materialien, wie Knochenschrauben, Schenkelhalsschrauben, Fusionskörpem, Schrauben für den so genannten Fixateur Externe, Schraubpfosten für Zahnimplantate oder einschraubbare künstliche Hüftgelenkpfannen, insbesondere zur Erzeugung von Neutrol- bzw. beliebigen Klemm- oder Freiwinkeln an den Gewindeflügeln.

14. Anwendung des Verfahrens gemäss einem oder mehreren der Ansprüche 1 bis 12 für die spanende Herstellung einschraubbarer künstlicher Hüftgelenkpfannen mit beliebiger äusserer Kontur des Schalenmantels, z.B. sphärisch, parasphärisch, konisch, konisch-sphärisch, parabolisch, usw., und einem auf dem Schalenmantel befindlichen Gewinde beliebiger Zahnstellung, z.B. neutral oder zum Pfannenpol gekippt, und beliebiger Steigung, z.B. konstanter oder variabler Steigung, mit einzelnen durch Spannuten voneinander getrennten Gewindeflügeln, zwecks Erzeugung so genannter Neutral- oder Freiwinkel an mindestens einer der Gewindezahnflächen.

15. Anwendung des Verfahrens gemäss einem oder mehreren der Ansprüche 1 bis 9 für die spanende Herstellung einschraubbarer künstlicher Hüftgelenkpfonnen mit beliebiger äusserer Kontur des Schalenmantels, z.B. sphärisch, parasphärisch, konisch, konisch-sphärisch, parabolisch, usw., und einem auf dem Schalenmantel befindlichen Gewinde beliebiger Zahnstellung, z.B. neutral oder zum Pfannenpol gekippt, und beliebiger Steigung, z.B. konstanter oder variabler Steigung, mit einzelnen durch Spannuten voneinander getrennten Gewindeflügeln, zwecks Erzeugung so genannter Schraub- oder Schraubenflächen an mindestens einer der Gewindezahnflächen.

16. Anwendung des Verfahrens gemäss einem oder mehreren der vorgenannten Ansprüche für die spanende Herstellung beliebiger Einschraubkörper zwecks Erzeugung gegenseitiger Verschwenkungen der Gewindeflügel.

17. Verfahren gemäss Anspruch 8, **dadurch gekennzeichnet, dass** die Unrundkontur als geschlossene Fläche mit sich wiederholenden Konturelementen gebildet ist.

18. Anwendung des Verfahrens gemäss Anspruch 17 für die Herstellung von Kreiskeil-Profilen bzw. Kreiskeilverbindungen.

## Claims

1. A turning method for unround turning on a programmable turning machine, whereby a workpiece is rotated in the clamping chuck of a machine spindle and thereby, with a tool on the workpiece, at least partially, defined unround contours, e.g. formed or constituted by transition elements, are produced by metal-cutting, **characterized in that** the turning is carried out in a hobbling manner, conveying the compound slide and the cutting tool in a synchronized manner to the spindle angle and producing the unround contours by means of programming jump functions by linking instruction sets with values for selected address parameters such as for example diameter (X), length (Z), gradient (F) or C (angle), whereby a sequence of address parameter values exhibiting a hobbling, i.e. a jump function is used at least for one of these address parameters in the sentence chain.

2. The method according to claim 1, **characterized in that** the parameter height (Y) is additionally used in the instruction sets.

3. The method according to any one of claims 1 or 2, **characterized in that** thread programming is used.

4. The method according to any one of claims 1, 2 or 3, **characterized in that** a hobbling sequence of address parameters is used for at least two of the aforementioned address parameters in the sentence chain.

5. The method according to any one of the preceding claims, **characterized in that** the sentence chain describes a rotation-symmetrical contour with a superimposed non-monotonical periodic sequence of increments.

6. The method according to any one of the preceding claims, **characterized in that**, for at least one of the address parameters, the increments formed between the address parameter values of the sentence chain are programmed as a hobbling sequence.

7. The method according to one or more of the preceding claims, **characterized in that** the discontinuous contour is produced by programming a pilgrim jump process, **in that** the tool is conveyed with a sequence of forward and backward movements, whereby one of the movements is larger than the other.

8. The method according to one or more of the preceding claims, **characterized in that** the unround or discontinuous contour on the workpiece is achieved by nesting at least two machining sequences, whereby a first contour element is produced by means of a first sequence, the next contour element is skipped, the following contour element is again produced, and the skipped contour element or elements are machined with a second sequence and the already machined contour elements are skipped.

9. The method according to one or more of the preceding claims for the metal-cutting production of discontinuously running contour elements projecting from an inclined or curved lateral surface, whereby the flank of the discontinuously running contour element is machined essentially with the side of the turning tool and the lateral surface is machined essentially with the tip of the turning tool, **characterized in that** the tip of the turning tool is guided on a path running essentially tangential to the lateral surface and the side of the turning tool produces the flank of the discontinuously running contour element by means of a programmed change in the tangential conveying speed and/or conveying direction.

10. The method according to one or more of the preceding claims, **characterized in that** the overshoot behaviour of the turning machine resulting from a jump instruction of the programming is used directly to produce discontinuous, unround or curved contours.

11. The method according to claim 10, **characterized in that** the overshoot behaviour of the turning machine is used for the direct production of cutting edges with clearance angles on thread segments or thread blades.

12. The method according to claim 11, **characterized in that** the cutting-edges are produced by at least partial milling-out of tapping grooves in the area of sections of the thread blades arising from an overshoot behaviour of the turning machine and the clearance angles thereby represent relics of the overshoot.

13. A use of the method according to one or more of claims 1 to 12 for the metal-cutting production of special threads, e.g. for yielding materials, on screw-in bodies, such as bone screws, leg-neck screws, fusion bodies, screws for the so-called external fixation, screw posts for tooth implants or threaded artificial hip-joint sockets, in particular for producing neutral or arbitrary jamming or clearance angles on the thread blades.

14. A use of the method according to one or more of claims 1 to 12 for the metal-cutting production of threaded artificial hip-joint sockets with arbitrary outer contour of the shell mantle, e.g. spherical, paraspherical, conical, conical-spherical, parabolical, etc., and a thread on the shell mantle with arbitrary tooth setting, e.g. neutral or tilted to the socket pole, and arbitrary pitch, e.g. constant or variable pitch, with individual thread teeth or blades separated from one another by tapping grooves, for the purpose of producing so-called neutral or clearance angles on at least one of the thread tooth surfaces.

15. A use of the method according to one or more of claims 1 to 9 for the metal-cutting production of threaded artificial hip-joint sockets with arbitrary outer contour of the shell mantle, e.g. spherical, paraspherical, conical, conical-spherical, parabolical, etc., and a thread on the shell mantle with arbitrary tooth setting, e.g. neutral or tilted to the socket pole, and arbitrary pitch, e.g. constant or variable pitch, with individual thread teeth or blades separated from one another by tapping grooves, for the purpose of producing so-called screw surfaces on at least one of the thread tooth surfaces.

16. A use of the method according to one or more of the preceding claims for the metal-cutting production of any screw-in bodies for the purpose of producing mutual swivelled settings of the thread teeth or blades.

17. The method according to claim 8, **characterized in that** the unround contour is formed as a closed surface with repeating contour elements.

18. A use of the method according to claim 17 for the production of circular-wedge profiles or circular-wedge joints.

## Revendications

1. Procédé de technique de tournage pour tournage non circulaire sur un tour programmable, une pièce étant en rotation dans le mandrin d'une broche de machine et des contours formés ou composés par exemple d'éléments géométriques de transition, non circulaires, au moins partiellement définis, étant réalisés par enlèvement de copeaux sur la pièce usinée au moyen d'un outil, **caractérisé en ce que** le tournage a lieu de manière chaotique en déplaçant le chariot à mouvements croisés avec l'outil d'enlèvement de copeaux de manière synchronisée avec l'angle de la broche et que les contours non circulaires sont réalisés au moyen d'une programmation à partir de fonctions de saut par rattachement d'articles d'instructions avec des valeurs pour les paramètres sélectionnés d'adresse comme par exemple le diamètre (X), la longueur (Z), la pente (F) ou l'angle (C), au moins une succession chaotique de valeurs, c'est-à-dire présentant une fonction de saut, de valeurs de paramètres d'adresse étant utilisée pour un de ces paramètres d'adresse.

2. Procédé selon la revendication 1, **caractérisé en ce que**, dans les articles d'instructions, le paramètre hauteur (Y) est utilisé en supplément.

3. Procédé selon une des revendications 1 ou 2, **caractérisé en ce qu**'une programmation de filetage est utilisée.

4. Procédé selon une des revendications 1, 2 ou 3, **caractérisé en ce que**, pour au moins deux des paramètres cités d'adresse dans la chaîne d'articles de programme, une suite chaotique de valeurs de paramètres d'adresse est utilisée.

5. Procédé selon une des revendications précédentes, **caractérisé en ce que** la chaîne d'articles de programme décrit un contour symétrique en rotation avec une succession périodique non monotone superposée d'incréments.

6. Procédé selon une des revendications précédentes, **caractérisé en ce que**, pour au moins un des paramètres d'adresse, les incréments formés entre les valeurs de paramètres d'adresse de la chaîne d'articles de programme sont programmés comme une suite chaotique.

7. Procédé selon une ou plusieurs des revendications précédentes, **caractérisé en ce que** la structure irrégulière est crée par la programmation d'un procédé à étapes de progression, en déplaçant l'outil avec une succession de mouvements vers l'avant et vers l'arrière, un des mouvements étant plus important que l'autre.

8. Procédé selon une ou plusieurs des revendications précédentes, **caractérisé en ce que** la structure non circulaire ou irrégulière est obtenue sur la pièce par inclusion d'au moins deux séquences d'usinage, un premier élément de contour étant réalisé au moyen d'une première séquence, le prochain élément de contour étant sauté, l'élément de contour suivant étant de nouveau réalisé, et le ou les éléments de contour sautés étant usinés par une seconde séquence et les éléments de contour déjà usinés étant alors sautés.

9. Procédé selon une ou plusieurs des revendications précédentes pour la fabrication par enlèvement de copeaux d'éléments de contour s'étendant irrégulièrement et dépassant d'une surface de chemise penchée ou courbée, le flanc de l'élément de contour s'étendant irrégulièrement étant usiné substantiellement avec le côté du burin de tournage et la surface de chemise étant usinée substantiellement avec la pointe du burin de tournage, **caractérisé en ce que** la pointe du burin de tournage est guidée sur une piste s'étendant substantiellement de manière tangentielle par rapport à la surface de la chemise et que le côté du burin de tournage réalise les flancs de l'élément de contour s'étendant irrégulièrement au moyen d'une modification programmée de la vitesse tangentielle de déplacement et/ou du sens de déplacement.

10. Procédé selon une ou plusieurs des revendications précédentes, **caractérisé en ce qu'**on tire directement parti du comportement de suroscillation du tour résultant d'une instruction de saut de la programmation pour réaliser des contours irréguliers, non circulaires ou courbés.

11. Procédé selon la revendication 10, **caractérisé en ce que** le comportement de suroscillation du tour est utilisé pour réaliser directement des arêtes coupantes avec des angles de dépouille sur des segments ou ailes à filetage.

12. Procédé selon la revendication 11, **caractérisé en ce que** les arêtes coupantes sont réalisées par un fraisage au moins partiel de dégagements dans la zone des sections résultant d'un comportement de suroscillation du tour des ailes à filetage et que les angles de dépouille représentent alors des reliquats de la suroscillation.

13. Application du procédé selon une ou plusieurs des revendications 1 à 12 pour la fabrication par enlèvement de copeaux de filetages spéciaux sur des corps à visser, par exemple pour des matières souples, comme des vis à os, des vis de col de fémur, des corps à fusion, des vis pour ce que l'on appelle un fixateur externe, des pitons à vis pour les implants dentaires ou des cotyles artificiels de hanches pouvant être vissés, notamment pour la fabrication d'angles neutres ou de n'importe quels angles de serrage ou de dépouille sur les ailes à filetage.

14. Application du procédé selon une ou plusieurs des revendications 1 à 12 pour la fabrication par enlèvement de copeaux de cotyles artificiels de hanches pouvant être vissés, avec n'importe quel contour extérieur de la chemise de la coque, par exemple sphérique, parasphérique, conique, sphéroconique, parabolique, etc., et avec un filetage positionné sur la chemise de la coque ayant n'importe quelle position de dents, par exemple neutre ou basculée vers le pôle du cotyle, et n'importe quelle pente, par exemple une pente constante ou variable, avec différentes ailes à filetage séparées les unes des autres par des dégagements, aux fins de créer ce que l'on appelle des angles neutres ou de dépouille sur au moins une des surfaces dentées du filetage.

15. Application du procédé selon une ou plusieurs des revendications 1 à 9 pour la fabrication par enlèvement de copeaux de cotyles artificiels de hanches pouvant être vissés, avec n'importe quel contour extérieur de la chemise de la coque, par exemple sphérique, parasphérique, conique, sphéroconique, parabolique, etc., et avec un filetage positionné sur la chemise de la coque ayant n'importe quelle position de dents, par exemple neutre ou basculée vers le pôle du cotyle, et n'importe quelle pente, par exemple une pente constante ou variable, avec différentes ailes à filetage séparées les unes des autres par des dégagements, aux fins de créer ce que l'on appelle des surfaces à vis ou de vissage sur au moins une des surfaces dentées du filetage.

16. Application du procédé selon une ou plusieurs des revendications précédentes pour la fabrication par enlèvement de copeaux de n'importe quels corps à visser aux fins de créer des torsions opposées des ailes à filetage.

17. Procédé selon la revendication 8, **caractérisé en ce que** le contour non circulaire est constitué comme une surface fermée avec des éléments de contour se répétant.

18. Application du procédé selon la revendication 17 pour la fabrication de profils à coins croisés ou de liaisons à coins croisés.
